# EUROPEAN PATENT SPECIFICATION

(11) **EP 4 530 285 B1**
(45) Date of publication and mention of the grant of the patent: **22.04.2026**
(21) Application number: 23206370.1
(22) Date of filing: 27.10.2023
(51) Int. Cl.: C07D 251/56, C07D 251/60, C07D 251/62, C07C 273/12, B01J 19/00, B01J 19/24

(54) **PROCESS FOR THE PRODUCTION OF MELAMINE**
VERFAHREN ZUR HERSTELLUNG VON MELAMIN
PROCÉDÉ DE PRODUCTION DE MÉLAMINE

(30) Priority: 26.09.2023 IT 202300019557
(43) Date of publication of application: 02.04.2025
(73) Proprietor: Eurotecnica Contractors and Engineers S.p.A., 20159 Milano (IT)
(72) Inventor: DE AMICIS, Alberto, San Donato Milanese (MI) (IT); DI RUOCCO, Giuseppe, Lecco (LC) (IT); SANTUCCI, Roberto, Gorla Maggiore (VA) (IT); RABAIOLI, Matteo, Casirate D Adda (BG) (IT); BOGOTTO, Mattia, Tradate (VA) (IT); PETRILLO, Francesca, Milano (MI) (IT)
(74) Representative: Bird & Bird Società tra Avvocati S.r.l.

(56) References cited:
- EP-A1- 2 094 652
- EP-B1- 2 094 652

## Description

The present invention relates to a process for the production of melamine and the relative plant.

There are essentially two types of processes for the production of melamine currently applied industrially, low-pressure processes (2-10 bar) with catalytic synthesis and non-catalytic high-pressure processes (> 70 bar).

In both processes, melamine is produced starting from urea according to the following overall reaction:

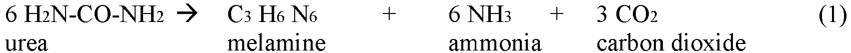

NH₃ and CO₂ are a product of the conversion reaction of urea into melamine and this stream leaving the reactor in gaseous form is called off-gas.

In so-called integrated processes for the synthesis of melamine and urea, the off-gas stream is recycled to the process for the production of urea wherein NH₃ and CO₂ are converted into urea according to the following two reactions in series:

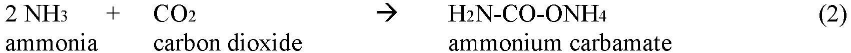

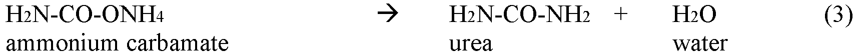

One of the most widespread industrial processes for the production of melamine is based on the pyrolysis of urea at high pressure and is described in the state of the art by US patents 7,125,992 and EP2385043 in the name of the same Applicant.

The state of the art of the processes that produce urea from NH₃ and CO₂ is described in literature in various publications, among the most authoritative publications, volume 37, chapter: "Urea" pages. 658-683 of Ullmann's Encyclopedia of Industria Chemistry, 7th edition revision, should be considered.

The integration of the urea process with the melamine process is widely described in the prior art, such as, for example, in EP2094652.

As described in the prior art, when the urea process is integrated with the melamine process, part of the urea produced by the urea process is used for the production of melamine and the off-gas stream generated by the melamine synthesis is recycled to the urea process for reconverting the ammonia and carbon dioxide contained in the off-gas stream back into urea.

According to the state of the art, the integration of the melamine process with the urea process mainly consists in identifying a section or apparatus, existing or new, of the urea plant, into which the off-gas stream coming from the melamine process is to be injected, with the aim of minimizing or cancelling the negative effects due to the recycling of the off-gas stream.

According to the state of the art, the integration is advantageous as, by reconverting the off-gas into urea, it allows the production of melamine by consuming urea in stoichiometric quantities according to reaction (1), i.e. producing melamine with a consumption of three moles of urea per mole of melamine, which equals 1.4tonU/tonM.

This integration, however, involves a series of technical problems: a first problem is that, when the two processes are integrated, the melamine process influences the operating capacity of the urea process with an increase in recycled streams and consequently an increase in energy consumption.

This problem is due to the fact that the off-gas stream represents an additional supply stream of the reagents (NH₃ and CO₂) to the urea process, and as such, the off-gas stream influences the load of the urea plant and the quantity of urea produced. When the melamine process is integrated with the urea process, the off-gas stream from the melamine process is, in fact, added as a supply stream to the two typical supply streams of the urea process which are a stream of liquid NH₃ and a gaseous stream of CO₂. A variation in terms of flow-rate, pressure or temperature of the off-gas stream coming from the melamine process consequently leads to a variation in the operating conditions of the urea process.

If, for example, the off-gas stream recycled to the urea process has a change in the flow-rate due to the trend of the melamine process, the urea process must compensate by modifying the flow-rate of the other two supply streams NH₃ and CO₂, in order to keep the production of urea constant. Furthermore, if the pressure of the off-gas stream decreases, the section of the urea plant receiving the off-gas stream must decrease its operating pressure to allow the off-gas stream to enter the urea process. If, on the other hand, it is the temperature of the off-gas stream from the melamine process that varies, the urea process must vary the other streams entering the section where the off-gas stream is recycled in order to maintain the required operating conditions. Any operational variation in the melamine plant is consequently reflected in an operational variation in the urea plant with the problem of running the urea plant in a non-optimal manner compared to the operating capacity guaranteed by feeding only the two streams of liquid NH₃ and gaseous CO₂ and consequently with an increase in the recycled streams and energy consumption.

Based on what has already been specified, the addition of a third reagent supply stream (i.e. the off-gas stream) inevitably leads to a reduction in gaseous CO₂ and liquid NH₃ fed to the urea process to counterbalance the CO₂ and NH₃ fed as off-gas and maintain the production capacity of the urea process.

Furthermore, in urea processes that use CO₂ as a stripping agent in the high-pressure decomposer (CO₂ stripping process), a reduction in the quantity of gaseous CO₂ in the feed leads to a reduction in the stripping efficiency which must be compensated by increasing the steam and energy consumption.

A second problem is the loss of efficiency of the urea process when it is integrated with a melamine process, particularly when the off-gas stream is recycled in the form of liquid carbamate. The water used for the condensation of the off-gas stream to carbamate has a negative effect on the conversion of the reagents into urea inside the reactor, with a consequent increase in the recycled streams and therefore in the energy consumption per ton of urea produced.

A further problem is that when a urea process already exists and is integrated with a melamine process, the integration may involve stopping the urea process to allow, for example, the off-gas stream to be connected with the urea reactor or with one of the sections downstream of the urea synthesis section. If, in fact, the integration also requires an increase in the production capacity (capacitive revamping) of the urea plant, the integration cannot be carried out during the scheduled shutdowns of the urea plant. In this case, without any doubt, stopping the urea process for the time necessary for carrying out the integration with the new melamine process represents a significant economic loss due to the lack of urea production.

The integration of an existing urea process with a new melamine process may in fact require the need for modifying the urea process in order to increase its production capacity (capacitive revamping), when the same is not sufficient for reconverting the additional stream of reagents recycled with the off-gas stream into urea, and reducing the flow-rate of the gaseous CO₂ stream and the liquid NH₃ stream and consequently reducing the urea produced, is not required. Capacitive revamping involves a higher cost than simply connecting the two processes as it requires the addition or replacement of equipment and therefore longer stoppage times. Capacitive revamping due to the integration of the existing urea process with the melamine process can lead to a significant economic loss due to the lack of urea production and, in some cases, the cost of lost production could make the implementation of a new melamine process unprofitable .

In any case, an integrated urea/melamine process, such as those described in the state of the art, is simply a process wherein the urea synthesis process substantially continues to operate as a non-integrated urea synthesis process and the melamine synthesis process continues to operate as a non-integrated melamine synthesis process: so-called "integration" means that a part of the urea synthesized in the urea process is sent to the "integrated" melamine process which uses it as a raw material; the off-gas deriving from the melamine synthesis process is sent to the "integrated" urea synthesis process as raw material.

For the rest, the two processes/plants essentially continue to function independently of each other.

The present invention aims to overcome the disadvantages of the known art previously indicated. The objective of the present invention is in fact an innovative process scheme for the production of melamine (Stand-Alone Melamine Process) which does not need to be integrated with the urea process and which allows the problems listed above to be overcome.

The present invention relates to a process for the production of melamine by means of a high-pressure urea pyrolysis reaction, comprising the following operating steps:
a) an off-gas condensation step (OGC), carried out at a pressure ranging from 70 to 220 bar, preferably 80-150 bar, to which the following streams are fed
   i) a gaseous stream of anhydrous off-gas 24 comprising NH₃ and CO₂, coming from a scrubbing step of the off-gas OGQ with molten urea, a stream 23 comprising NH₃, CO₂ and melamine vapours, produced as a gaseous effluent from the urea pyrolysis reaction in the reaction section R and the post-reaction and ammonia stripping section PR of the melamine synthesis process,
   ii) a liquid stream 17 comprising NH₃, CO₂ and H₂O, coming from an ammonia recovery step AR;
   iii) a gaseous stream 14 comprising NH₃, CO₂ and H₂O coming from a process-water treatment step PWT and
   iv) a gaseous stream of off-gas 27 comprising NH₃, CO₂ and H₂O, coming from a high-pressure off-gas decomposition step HPD;
   said streams i.-iv. being fed to the off-gas condensation step OGC at the same pressure at which the condensation step OGC is carried out, and obtaining a liquid stream 25 comprising NH₃, CO₂ and H₂O;
b) a conversion step OGR to which said liquid stream 25 comprising NH₃, CO₂ and H₂O obtained at the end of said step a) is fed, obtaining a first stream 26 comprising urea, ammonia, carbamate and H₂O;
c) a high-pressure decomposition step HPD to which the first stream 26 comprising urea, ammonia, carbamate and H₂O obtained at the end of the conversion step b) is fed, obtaining the stream iv) 27 fed to the condensation step a) and a second stream 28 comprising urea, carbamate and H₂O;
d) a medium-pressure decomposition step MPD to which the second stream (28) comprising urea, carbamate and H₂O obtained at the end of the high-pressure decomposition step c) HPD is fed, obtaining an off- gas stream 29 comprising NH₃, CO₂ and water which is fed to the ammonia recovery step AR, and a third stream 30 comprising urea, carbamate and H₂O;
e) a low-pressure decomposition step LPD to which the third stream 30 comprising urea, carbamate and H₂O obtained at the end of the medium-pressure decomposition step d) MPD is fed, obtaining an off-gas stream 31 comprising NH₃, CO₂ and water, which is fed to the process-water treatment step PWT, and a fourth stream 32 comprising urea and H₂O;
f) a urea concentration step CON to which the fourth stream 32 comprising urea and H₂O obtained at the end of the low-pressure decomposition step e) LPD is fed, obtaining a stream of concentrated urea 33 which is fed to the off-gas scrubbing step OGQ with molten urea and a stream of H₂O 34 which is fed to the process-water treatment step PWT.

The stream 23, comprising NH₃, CO₂ and melamine vapours, fed to the scrubbing step with urea OGQ, can comprise the gaseous stream 21 alone, comprising NH₃, CO₂ and melamine vapours, obtained together with a liquid stream 3 of raw melamine comprising melamine, unreacted urea, oxidized intermediate products of OAT pyrolysis and deammoniation and condensation products of melamine (polycondensates), from the separation of a biphasic liquid-gaseous effluent produced by the urea pyrolysis reaction R.

Alternatively, the stream 23, comprising NH₃, CO₂ and melamine vapours, fed to the scrubbing step with urea OGQ, can comprise said first stream of anhydrous off-gas 21 and a second stream 22 of anhydrous off-gas comprising NH₃, CO₂ and melamine vapour, obtained by putting said liquid stream 3 of raw melamine in contact, in a post-reaction and stripping with ammonia step PR, with a stream of gaseous NH₃, anhydrous and superheated under pressure 16P, to form a liquid stream 4 of raw melamine depleted of CO₂ and said second stream 22 of anhydrous off-gas.

Off-gas streams 27, 29 and 31 are mainly composed of NH₃, CO₂ and H₂O and may further comprise passivation air.

In the process according to the present invention, said liquid stream 4 of raw melamine depleted of CO₂, before being subjected to a purification step P and a crystallization step C by cooling, is subjected to a quench-ammonolysis treatment QAL through contact with at least one aqueous ammonia stream 12, with the formation of an aqueous ammonia stream 5 comprising purified melamine substantially free of polycondensates, said quench-ammonolysis treatment QAL being preferably carried out by contact with said aqueous ammonia stream 12 coming from the process-water treatment step PWT and with a pure ammonia stream 15 coming from the ammonia recovery step AR.

Said aqueous ammonia stream (5), comprising purified melamine substantially free of polycondensates, is fed to the purification step P from which a solution of purified melamine 6 is obtained, fed to the cold crystallization step C, from which a suspension of melamine 7 is obtained, then fed to a separation step S from which a stream of pure melamine 200 and a stream of mother liquor 9 are obtained, said mother liquor stream 9 comprising H₂O, traces of melamine and OATs being partly fed to the process-water treatment step PWT, where it is put in contact with the aqueous stream 34 coming from step f) and with the off-gas stream 31 comprising NH₃, CO₂ and water coming from the low-pressure decomposition LPD step e), obtaining at the end of the process-water treatment step PWT, a stream of pure water 300, the liquid stream 12 comprising ammonia and water which is sent to the quench-ammonolysis step QAL, a liquid stream 13 comprising water, ammonia and CO₂ which is sent to the ammonia recovery step AR and the gaseous stream 14 comprising water, ammonia and CO₂ which is sent to the off-gas condensation section OGC.

More specifically, in the process-water treatment step PWT, the stream of mother liquor 9, comprising H₂O, traces of melamine and OATs is subjected to a decomposition treatment by hydrolysis at a temperature ranging from 270°C to 300°C, with the formation of a gaseous stream 14, comprising NH₃, CO₂ and H₂O, and a liquid stream which is subjected to a stripping treatment in order to obtain a liquid stream 300 of pure water.

Said ammonia recovery step AR preferably removes from the liquid stream 13 comprising water, ammonia and CO₂ coming from the process-water treatment step PWT and from the stream of off-gas 29 coming from the medium-pressure decomposition step MPD, at least a part of the ammonia contained therein, forming a liquid stream 17 comprising NH₃, CO₂ and water which is sent to step a) for the condensation of the off-gas OGC and a stream of pure ammonia which is sent partly 15 to the quench-ammonolysis step QAL, partly 16R to the pyrolysis reaction R and partly 16P, to the post-reaction and stripping with ammonia step PR. The ammonia that is sent to the pyrolysis reaction R and to the post-reaction and stripping step PR is preferably gaseous ammonia, anhydrous and superheated under pressure.

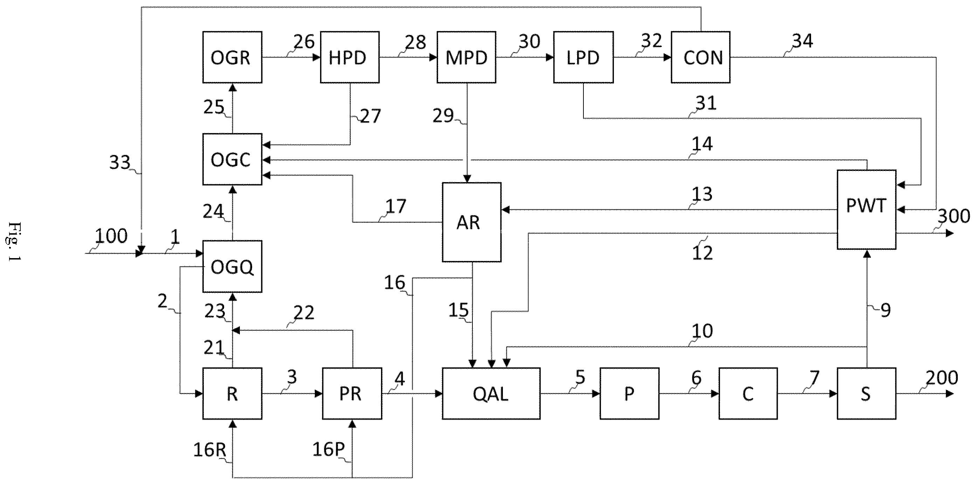

In an embodiment of the process according to the present invention, (represented in figure 1) the urea fed to the off-gas scrubbing step OGQ is concentrated urea 33 coming from the urea concentration step f) CON, possibly with the addition of a stream 100 of molten urea from the battery limits, the two streams 21, 22 of anhydrous off-gas comprising NH₃, CO₂ and melamine vapours coming from the reaction step R and from the post-reaction and stripping with ammonia step PR, being fed, together or separately, to said off-gas scrubbing step OGQ with urea, obtaining a stream 24 of scrubbed off-gas which is sent to step a) for the condensation of the off-gas OGC and a stream of urea 2 comprising the melamine removed from the off-gas stream 23, which is recycled to the reaction step R.

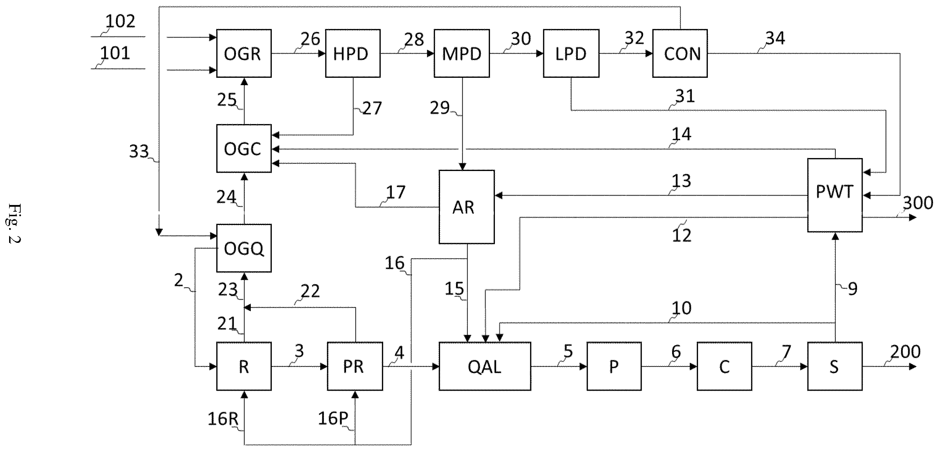

In a different embodiment of the process according to the present invention, (represented in figure 2), the urea fed to the off-gas scrubbing step OGQ is only concentrated urea 33 coming from the urea concentration step f) CON, a stream of liquid ammonia 101 and a stream of gaseous CO₂ 102 being fed to the conversion step OGR to be put in contact with the liquid carbamate stream 25 comprising ammonia, CO₂ and water coming from the off-gas condensation OGC step a) to obtain the first stream 26 containing urea, ammonia, carbamate and water, in turn sent to the high-pressure decomposition step b) HPD.

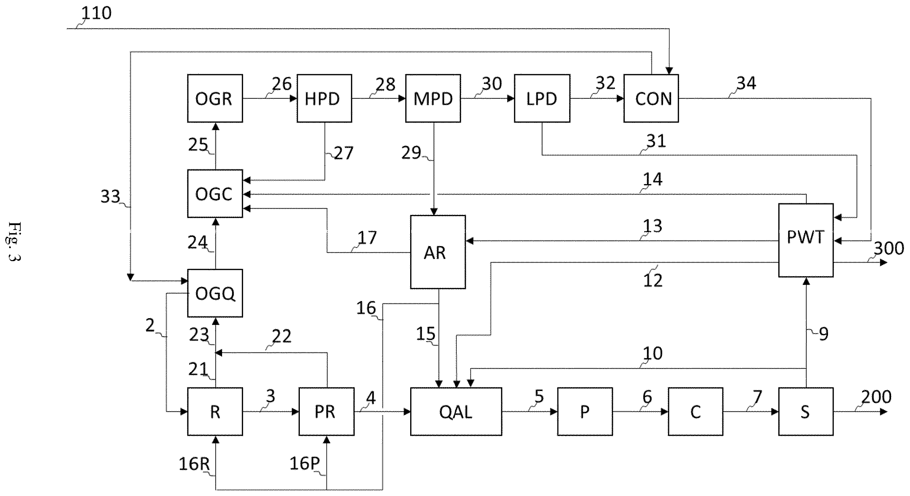

In a further embodiment of the process according to the present invention, (represented in figure 3), the urea concentration step f) CON receives a stream 110 of urea solution from the battery limits from outside the plant, and supplies a stream of concentrated urea 33 to the off-gas scrubbing step OGQ, whereas a recycling stream 2 of urea comprising the melamine removed from the off-gas stream 23 coming from the off-gas scrubbing step OGQ is fed to the reaction step R.

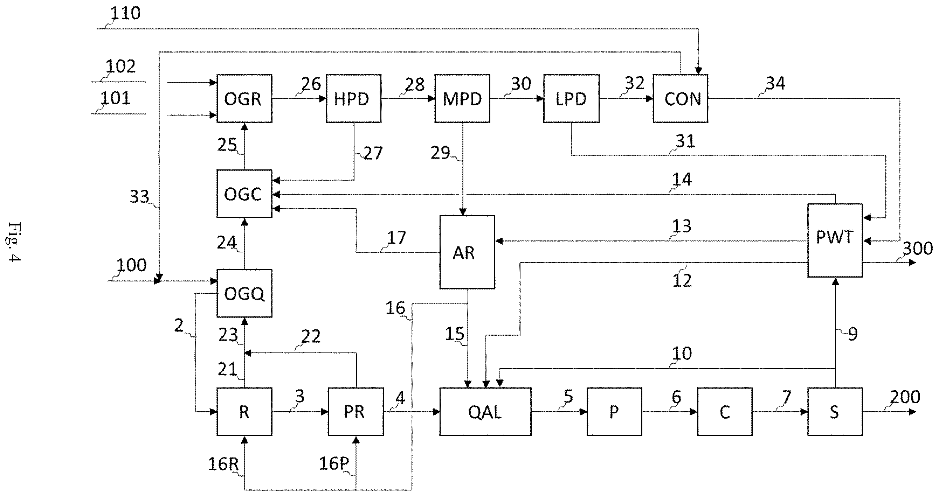

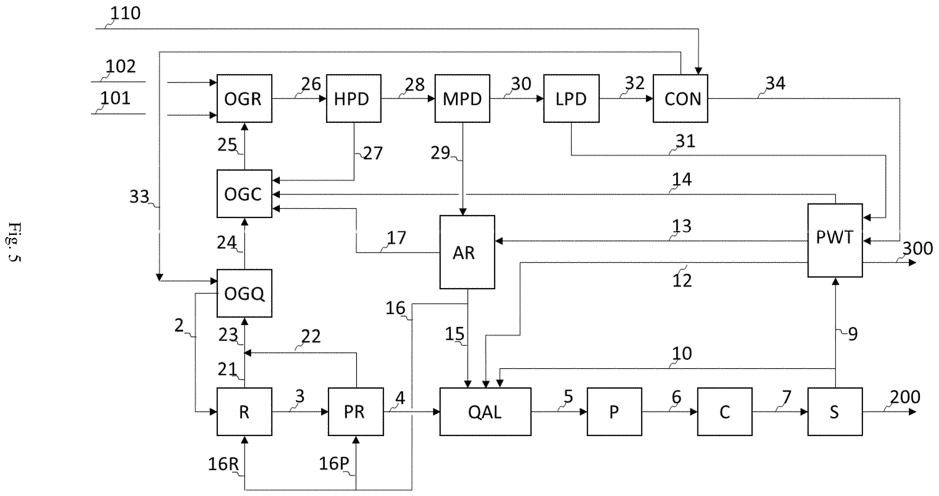

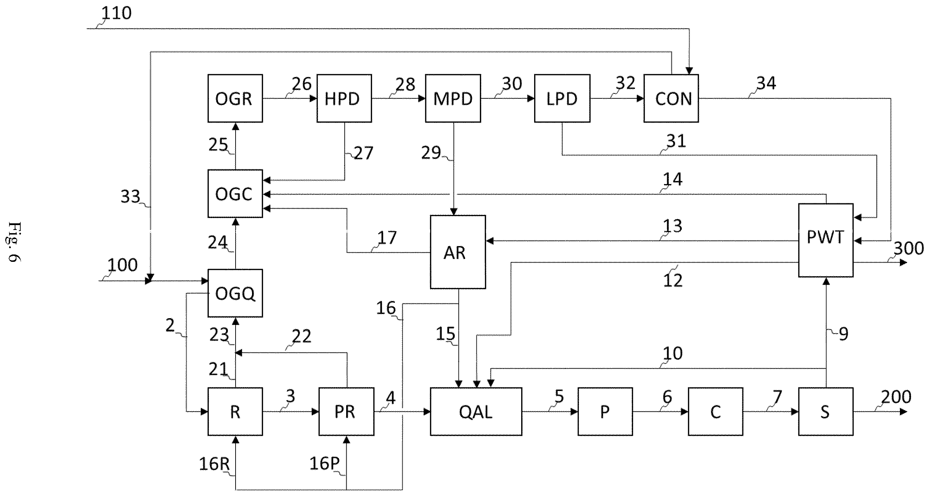

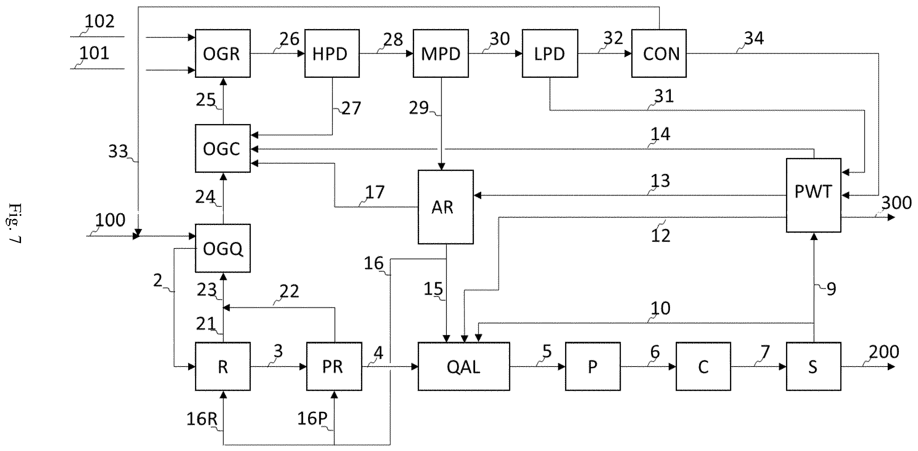

The three embodiments represented in figures 1-3 can therefore be effected as alternatives or combined in all possible ways as represented in figures 4-7.

In the present description, when the definition "high pressure" is used, it refers to a pressure range that varies from 70 to 220 bar, preferably from 80 to 150 bar; the definition "medium pressure" refers to a pressure range that varies from 8 to 30 bar, the definition "low pressure" refers to a pressure range that varies from 1 to 8 bar.

The present invention further relates to a plant for the production of melamine as defined in claims 9-14.

In the plant according to the present invention, there are suitable pumps for correctly moving the fluid streams when necessary based on pressure differences, and there are also suitable heat exchangers when necessary based on temperature differences. These devices are not shown in the figures or specified in the description of the various lines as they are of common knowledge to skilled persons in the field.

In the plant according to the present invention the sections OGC and OGQ operate at the same pressure as the section R and, as the section OGC is connected to the section HPD, the section HPD operates at the same pressure as the section R: consequently stream 14 fed to the section OGC is brought to the same pressure.

The present invention is described hereunder with reference to the following schematic figures.

In the figures attached to the present invention,
- figure 1 is a block diagram of the melamine production process according to a first embodiment of the present invention;
- figures 2-7 are block diagrams of the melamine production process according to further embodiments of the present invention.

In the following description, for illustrating the figures, identical reference numbers are used for indicating elements with the same function. Furthermore, for clarity of illustration, some numerical references may not be repeated in all the figures.

The different characteristics in the different embodiments can be combined together as desired according to the previous description, should the advantages resulting specifically from a particular combination be used.

A first embodiment of the Stand-Alone Melamine Process, object of the present invention, is illustrated in the block diagram of figure 1 and comprises:
a scrubbing section of the off-gas OGQ for recovering the melamine vapour contained in the anhydrous off-gas coming from the Reaction section R and the Post-Reaction section PR. The anhydrous off-gas streams 21 and 22 coming from the sections R and PR are subjected, together or separately, to a melamine recovery treatment by scrubbing with urea, in a single off-gas scrubbing section OGQ or in separate scrubbing sections (not shown in figure 1).

The second stream 22 of anhydrous off-gas coming from the Post-Reaction section is preferably combined with the first stream 21 of anhydrous off-gas coming from the reaction section, forming a single stream 23 of anhydrous off-gas which is subjected to the same treatment in a single scrubbing section of the off-gas with urea OGQ. The section OGQ operates at approximately the same pressure as the Reaction R and Post-Reaction PR sections, whereas the scrubbing temperature varies within the range of 140°C to 250°C. In the scrubbing with urea, the melamine contained in the anhydrous off-gas is removed and brought into solution/suspension in the urea. The stream of urea 1 fed to the off-gas scrubbing section OGQ is obtained from the combination of the molten urea stream from the battery limits 100 and the recycled concentrated urea stream 33, coming from the urea concentration section CON. The urea stream 2 exiting from the off-gas scrubbing section OGQ is fed to the Reaction section R, whereas the anhydrous off-gas stream 24 is fed to the off-gas condensation section OGC.

The Reaction section R comprises a pyrolysis reactor that operates continuously and in which a suitable heating system provides the reagent system with the necessary reaction calories, maintaining the temperature within the range of 360°C to 420°C. The reaction pressure is kept at a value higher than 70 bar, preferably within the range of 70 to 220 bar, more preferably within the range of 80 to 150 bar. A stream of gaseous NH₃, anhydrous and superheated under pressure 16R, coming from the ammonia recovery section AR is also preferably introduced into the reactor together, with the urea.

The product of the urea pyrolysis reaction, consisting of a two-phase effluent comprising a gaseous phase 21 and a liquid phase 3, is subjected to subsequent treatments. The liquid phase 3, exiting from the Pyrolysis Reactor R, containing melamine, unreacted urea, oxidized intermediate products of OAT pyrolysis and melamine deammoniation and condensation products (polycondensates), is sent to a subsequent reaction step in a Post-Reactor PR which operates under temperature and pressure conditions substantially the same as those of the Pyrolysis Reactor R and into which a stream of gaseous, anhydrous and superheated NH₃ under pressure 16P is fed, in order to eliminate the dissolved CO₂ and complete the urea pyrolysis reaction and transform most of the OATs into melamine, obtaining a liquid phase of melamine 4 and a second gaseous phase 22.

The liquid phase of melamine exiting from the Post-Reactor 4 is brought into aqueous solution in a quench-ammonolysis section QAL, in the presence of NH₃, fed as pure NH₃ by means of a stream 15 coming from the ammonia recovery section AR and as ammonia in solution by means of a stream 12 coming from the water treatment section PWT, the whole system being kept under controlled conditions of temperature and residence time, preferably at a temperature ranging from 160°C to 180°C, more preferably from 170°C to 172° C, at a pressure ranging from 25 to 45 bar, more preferably equal to 30 bar, for a time ranging from 30 to 60 minutes, more preferably equal to 45 minutes, obtaining a solution substantially free of polycondensates 5 which is brought to a purification section P to obtain a solution free of impurities 6 which is then subjected to crystallization in the crystallization section C, to obtain a melamine suspension 7 which is then fed to the separation section S to give high-purity melamine 200. A part of the mother liquor 10 containing melamine and small quantities of OATs is recycled from the section S, without any treatment, to the quench-ammonolysis section QAL.

The remaining part of the mother liquor 9 is treated in a process-water treatment section PWT. Said section PWT also receives an off gas stream 31 from the low-pressure decomposition section LPD and a liquid stream 34 of process water coming from the urea concentration section CON. A liquid stream containing water and ammonia 12 is obtained, through decomposition and distillation, from the process-water treatment section PWT, which is sent to the quench-ammonolysis section QAL, together with a liquid stream 13 containing water, NH₃ and CO₂ which is sent to the ammonia recovery AR section, a gas stream 14 containing NH₃, CO₂ and water sent to the off-gas condensation section OGC and a stream of purified process water 300 sent to the battery limits.

The ammonia recovery section AR, in addition to receiving the liquid stream 13 from the process-water treatment section PWT, also receives the off-gas gaseous stream 29 from the medium-pressure decomposition section MPD. In the ammonia recovery section AR, a stream of pure ammonia is obtained, by distillation, partly recycled to the quench-ammonolysis section QAL as stream 15, partly recycled to the reaction section R as stream 16R and partly recycled to the Post-reactor RP as stream 16P. A second liquid stream 17 is obtained from the ammonia recovery section AR, at the outlet, containing NH₃, CO₂ and water sent to the off-gas condensation section OGC.

Step a) of the process according to the present invention is carried out in the off-gas condensation section OGC which has an operating pressure equal to or slightly lower than the off-gas scrubbing section OGQ, which in turn has an operating pressure equal to or slightly lower than the reaction section R, and has an operating temperature such as to obtain the total condensation of the incoming gaseous streams. The off-gas condensation section OGC receives three gaseous streams, the off-gas stream 24 from the off-gas scrubbing section OGQ, the stream 14, comprising NH₃, CO₂ and water, from the process-water treatment section PWT and the off-gas stream 27 from the high-pressure decomposition section. Furthermore, the section OGC receives a liquid stream 17, comprising NH₃, CO₂ and water, from the ammonia recovery section AR. The condensation heat of the three gaseous streams is recovered by generating steam which is used within the Stand-Alone Melamine Process. The liquid stream 25 exiting from the section OGC comprising NH₃, CO₂ and water is pumped to the conversion section OGR, where step b) of the process according to the present invention takes place. The liquid stream 25 can be possibly pre-heated to improve the conversion of the off-gas in step b) of the process according to the present invention.

The off-gas conversion section operates at a pressure within the range of 120 bar to 250 bar, preferably at a pressure within the range of 140bar to 220 bar. The operating temperature of the section OGR varies within the range of 150°C to 250°C, preferably within the range of 170°C to 220°C and the molar ratio NH₃/CO₂ is equal to 2.2-5, preferably equal to 2.5- 4.5 mole/mole.

The carbamate fed with stream 25, comprising NH₃, CO₂ and water, is partially converted into urea inside the section OGR. The stream 26 exiting from the section OGR contains urea, ammonia, unreacted carbamate and water. This stream 26 is sent to step c) of the process according to the present invention in the high-pressure decomposition section HPD where the liquid carbamate is decomposed into NH₃ and CO₂ in an exchanger to which heat is supplied by steam. The section HPD operates at the same pressure as the section OGC or slightly higher, and at an operating temperature within the range of 150 to 250°C, preferably 170°C to 220°C. A gaseous stream 27 is formed at the outlet of the section HPD, which is recycled to the section OGC, together with a liquid stream 28 containing urea, water and also a part of non-decomposed carbamate, which is sent to step d) of the process according to the present invention in the medium-pressure decomposition section MPD. The section MPD operates at a pressure ranging from 8 to 30 bar, preferably at a pressure within the range of 12 to 24 bar and at an operating temperature within the range of 140°C to 180°C, preferably 150° C to 170°C. In the section MPD, part of the carbamate contained in stream 28 is decomposed, the necessary heat is supplied by steam, and a liquid stream 30 containing urea, water and a part of carbamate is obtained, which is sent to step e) of the process according to the present invention in the low-pressure decomposition section LPD together with an off-gas gaseous stream 29 sent to the ammonia recovery section AR. The section LPD operates at a pressure within the range of 1 to 8 bar, preferably at a pressure within the range of 2 to 6 bar and at an operating temperature within the range of 130°C to 170°C, preferably 140° C to 160°C. In the section LPD, the carbamate contained in the stream 30 is decomposed, the necessary heat is supplied by steam, a liquid stream 32 is obtained, mainly comprising urea and water, sent to step f) of the process according to the present invention in the urea concentration section CON and a gaseous stream 31 sent to the process-water treatment section PWT. The urea solution is concentrated in the section CON, obtaining a urea stream 33 which is recycled to the section OGQ and a process-water stream 34, mainly comprising water and a low concentration of urea, NH₃ and CO₂ which is sent and treated in the section PWT.

In the above-mentioned liquid stream 32, comprising mainly urea and water, the term "prevalently" means that the stream contains approximately 98-99% by weight of urea and water, the remaining percentage being composed of traces of ammonia and CO₂. In the process-water stream 34, comprising mainly water and a low concentration of urea, NH₃ and CO₂, the term "prevalently" means that the stream contains approximately 98-99% by weight of water.

A second embodiment of the Stand-Alone Melamine Process, object of the present invention, is illustrated in the block diagram of figure 2 and differs from the first embodiment in that the urea fed to the off-gas scrubbing step is only the stream of recycled concentrated urea 33 coming from the urea concentration step f) which is effected in the concentration section CON, whereas a liquid ammonia stream 101 and a gaseous CO₂ stream 102 are fed to the conversion step in the section OGR to be put in contact with the liquid stream comprising ammonia, CO₂ and water coming from the off-gas condensation step a) in the section OGC to obtain the first stream containing urea, carbamate and water to be sent to step c) in the high-pressure decomposition section HPD.

In a third embodiment of the process according to the present invention, (represented in figure 3), the urea concentration step f) CON receives a stream 110 of urea solution from the battery limits from outside the system, and feeds a concentrated urea stream 33 to the off-gas scrubbing step OGQ, whereas a recycling stream of urea 2 comprising the melamine removed from the off-gas stream 23 coming from the off-gas scrubbing step OGQ is fed to the reaction step R.

The three embodiments illustrated in the block diagrams of figures 1-3 can therefore be effected as alternatives or they can constitute embodiments of the process according to the present invention, combining them in all possible ways as represented in figures 4-7.

The present invention as described above relates to a non-catalytic high-pressure melamine production process which does not need to be integrated with a urea process, as the off-gas produced from the synthesis of urea to melamine are converted back into urea within the same process for the production of melamine, thus solving the problems dependent on integration with a urea process.

The process for the production of melamine, object of the present invention, has an innovative element compared to the known art of being a scheme which uses some sections in common both in the steps of the melamine production process relating to the production, treatment and purification of the melamine streams and in the steps of the melamine production process according to the present invention relating to the treatment of the off-gas: these common sections have revealed surprising positive effects by solving the problems listed above.

In the process scheme according to the present invention, in fact, the process-water treatment section PWT, the ammonia recovery section AR and the off-gas condensation section OGC are common sections, i.e. they receive and return streams to the process sections for the production of melamine and the sections of the process for the treatment of off-gas and the production of urea.

The off-gas condensation section OGC has the purpose of condensing the high-pressure off-gas gaseous streams coming from the different steps of the process for the production of melamine according to the present invention and using the condensation heat for generating steam, in turn used in the melamine production process. The section OGC receives the anhydrous off-gas from the section OGQ, it receives the off-gas generated by the decomposition of the carbamate (a stream containing NH₃, CO₂ and H₂O) from the section HPD, it receives an off-gas stream 14 from the common section PWT and a liquid stream of carbamate 17 from the common section AR. The section OGC returns a liquid stream of carbamate fed to the conversion section OGR to convert some of the carbamate back to urea.

The ammonia recovery section AR has the purpose of recovering pure ammonia and recycling it to the appropriate sections. The section AR receives the off-gas from the section MPD, it receives a liquid carbamate stream 13 from the section PWT and returns a liquid carbamate stream 17 to the section OGC and a stream of pure ammonia sent partly as stream 15 to the section QAL and partly as streams 16R, 16P to the reaction section R and to the post-reaction and ammonia stripping section PR.

The process-water treatment section PWT has the purpose of treating all the process water and recycling it to the appropriate sections. The section PWT receives the mother liquor from the section S with stream 9, the process water from the urea concentration CON with stream 34, the off-gas from the low-pressure decomposition section with stream 31 and returns a high-pressure off-gas stream 14 to the section OGC, a liquid stream 12 of NH₃ and water to the section QAL and a liquid stream of carbamate 13 to the section AR.

It should also be pointed out that, unlike the sections HPD, MPD and LPD of the integrated melamine/urea processes of the state of the art, each of which comprises a decomposer (heater) and a condenser, the sections HPD, MPD and LPD of the process for the production of melamine according to the present invention require only the presence of a heater with considerable advantages in terms of installation costs and consumption. Condensers are not necessary thanks to the particular process scheme according to the present invention which involves the use of sections OGC, AR and PWT in common for different steps of the process.

A further advantage of the process according to the present invention and the relative plant is that the entire system is managed by a single operating control unit.

The following example represents an embodiment of the present invention.

### EXAMPLE 1

In a melamine plant with a nominal capacity of 40,000 t/y, 16.0 t/h of molten urea are sent to the off-gas scrubbing section OGQ to effect the scrubbing of the off-gas stream of 13.0 t/h, coming from the reaction sections R and PR, in order to obtain a stream of anhydrous off-gas, free of melamine; the section OGQ operates at the same pressure as the reaction sections R and PR, 110 bar and at a temperature of 215°C.

The molten urea containing the melamine recovered from the section OGQ and a stream of gaseous NH₃ of 1.0 t/h are fed to the melamine reactor R; the reactor operates at 380°C and 110 bar.

11.8 t/h of anhydrous off-gas comprising melamine steam and 5.7 t/h of raw liquid melamine are separated from the reactor. This raw liquid melamine is treated under the same reactor conditions in a post reactor PR with 1.1 t/h of anhydrous gaseous NH₃ and superheated under pressure, obtaining 5.6 t/h of liquid melamine comprising only 0.02% by mass of dissolved CO₂; this stream also contains 3.4% by mass of polycondensates, 0.6% by mass of OATs and is substantially free of unreacted urea.

1.2 t/h of anhydrous off-gas comprising melamine steam is separated from the post-reactor PR, which combined with the off-gas exiting from the reactor R form a stream of 13.0 t/h comprising 3.8% by mass of melamine steam which, as indicated above, is sent to the off-gas scrubbing section OGQ.

The liquid melamine exiting from the PR is sent to the quench-ammonolyser QAL for purification in aqueous solution with NH₃, under the following operating conditions: 172°C, 30 bar and NH₃ concentration 14% by mass. The aqueous solution present in the quench-ammonolyser is obtained by recycling to QAL an aqueous stream coming from the section PWT of 53.9 t/h having a concentration of NH₃ at 15% by mass and a stream coming from the section AR of 0.2 t/h of pure ammonia.

67.1 t/h of an aqueous solution containing 8% by mass of melamine, 14% by mass of NH₃ and comprising approximately 4,000 ppm by mass of OATs and approximately 500 ppm by mass of polycondensates, leave the QAL quench-ammonolyser.

This solution is fed to the purification section P, comprising filters, from which a solution containing less than 100 ppm by mass of polycondensates exits; the purification section P operates under the same conditions as the section QAL (pressure 30 bar and temperature 172°C). The solution exiting from the section P is then sent to the crystallizer C, which operates at a temperature of 45 °C, a pressure of 0.5 bar and a pH of approximately 11.5.

The suspension exiting from the crystallizer C is sent to a solid/liquid separator (centrifuge) S, which separates the crystallization mother liquor from the high-purity melamine (titre over 99.9% by mass on dry matter).

The approximately 62.1 t/h of mother liquor exiting from S is divided into two streams. A stream of 7.4 t/h is recycled directly to the quench-ammonolysis section QAL, without undergoing any treatment; the remaining 54.7 t/h are sent to the process-water treatment section PWT.

The section PWT, in addition to receiving the stream of mother liquor, receives a stream of process water from the urea concentration section CON and an off-gas stream from the low-pressure decomposition section LPD. The purpose of the section PWT is to purify the process water by thermally decomposing urea, OATs and melamine contained in the incoming streams obtaining NH₃ and CO₂, together with pure water to be sent to the battery limit. A stream of purified process water of 2.1 t/h sent to the battery limit, a liquid stream of water and ammonia of 53.9 t/h recycled to the section QAL, a liquid stream of carbamate (NH₃, CO₂ and H₂O) of 3.7 t/h recycled to the section AR and a stream of gaseous off-gas of 0.9 t/h recycled to the section OGC, exit from the section PWT.

The section PWT comprises equipment for the thermal decomposition of urea, for the thermal decomposition of OATs and melamine at a temperature of 290°C and equipment for the distillation and recovery of ammonia which is thus recycled internally.

The ammonia recovery section AR has the purpose of distilling pure ammonia from the incoming streams and recycling it within the process. The ammonia recovery section AR, in addition to the carbamate stream from the section PWT, also receives an off-gas stream of 4.9 t/h from the medium-pressure decomposition section MPD. At the outlet of the section AR, there is a liquid stream of 2.3 t/h of pure ammonia and a liquid stream of 6.3 t/h of carbamate recycled to the section OGC.

The off-gas condensation section OGC has the purpose of condensing the incoming gaseous off-gas streams and using the condensation heat for generating steam and therefore recovering energy. The section OGC, in addition to receiving the off-gas stream from the section PWT and the liquid carbamate stream from the section AR, receives the gaseous off-gas stream of 10.1 t/h from the high-pressure decomposition section HPD and receives the gaseous off-gas stream of 12.5 t/h from the section OGQ. The section OGC has an operating pressure slightly lower than the sections OGQ and HPD from which it receives the off-gas, its operating conditions are 105 bar and 155°C. At the outlet of the section OGC, a stream of liquid carbamate of 29.8 t/h is obtained which is preheated and fed to the conversion section OGR where the carbamate is converted into urea according to reaction (3).

The section OGR operates at a pressure of 200 bar and a temperature of 190°C and a liquid stream is obtained at the outlet, having a concentration of 30% by mass of urea, containing unreacted carbamate and water. This stream is treated in the section HPD where part of the carbamate contained in the stream exiting from the section OGR is thermally decomposed at a pressure of 105 bar and at a temperature of 200°C obtaining a liquid stream of 19.7 t/h having a concentration of 45% urea by mass. The stream is further concentrated in the section MPD at a pressure of 18 bar and a temperature of 160°C, obtaining a liquid stream of 14.8 t/h at the outlet, containing 60% urea by mass. A further decomposition step at low pressure at 4 bar and a temperature of 145°C allows a liquid solution of 13.6 t/h to be obtained, containing water and urea with a mass concentration of 65% urea. This stream is concentrated in the concentration section CON operating at a pressure lower than atmospheric pressure and at a temperature of 136°C, from which a stream of concentrated urea of 8.9 t/h and a stream of process water of 4.7 t/h are obtained, sent to the section PWT. The stream of concentrated urea from the section CON is combined with a stream of molten urea of 7.1 t/h from the battery limit to form a stream of urea of 16 t/h fed to the off-gas scrubber section OGQ.

The example describes a melamine plant with a nominal capacity of 40,000 t/y which receives from the battery limit only a stream of molten urea of 7.1 t/h and which has, at the outlet, a stream of pure melamine of 5 t/h and a stream of pure water of 2.1 t/h.

The mass percentages indicated in this example are calculated with respect to the total mass of the stream or solution considered.

## Claims

1. A process for the production of melamine by means of a high-pressure urea pyrolysis reaction, comprising the following operating steps:
a) an off-gas condensation step (OGC), carried out at a pressure ranging from 70 to 220 bar, preferably 80-150 bar, to which the following streams are fed
i) a gaseous stream of anhydrous off-gas (24) comprising NH₃ and CO₂, coming from a scrubbing step of the off-gas (OGQ) with urea, a stream (23) comprising NH₃, CO₂ and melamine vapours, produced as a gaseous effluent from the urea pyrolysis reaction in the reaction section (R) and the post-reaction and ammonia stripping section (PR) of the melamine synthesis process,
ii) a liquid stream (17) comprising NH₃, CO₂ and H₂O, coming from an ammonia recovery step (AR);
iii) a gaseous stream (14) comprising NH₃, CO₂ and H₂O coming from a process-water treatment step (PWT) and
iv) a gaseous stream of off-gas (27) comprising NH₃, CO₂ and H₂O, coming from a high-pressure off-gas decomposition step (HPD);
said streams i.-iv. being fed to the off-gas condensation step (OGC) at the same pressure at which the condensation step (OGC) is carried out, and obtaining a liquid stream (25) comprising NH₃, CO₂ and H₂O;
b) a conversion step (OGR) to which said liquid stream (25) comprising NH₃, CO₂ and H₂O obtained at the end of said step a) is fed, obtaining a first stream (26) comprising urea, ammonia, carbamate and H₂O;
c) a high-pressure decomposition step (HPD) to which the first stream (26) comprising urea, ammonia, carbamate and H₂O obtained at the end of the conversion step b) is fed, obtaining the stream iv) (27) fed to the condensation step a) and a second stream (28) comprising urea, carbamate and H₂O;
d) a medium-pressure decomposition step (MPD) to which the second stream (28) comprising urea, carbamate and H₂O obtained at the end of the high-pressure decomposition step c) (HPD) is fed, obtaining an off- gas stream (29) comprising NH₃, CO₂ and water which is fed to the ammonia recovery step (AR), and a third stream (30) comprising urea, carbamate and H₂O;
e) a low-pressure decomposition step (LPD) to which the third stream (30) comprising urea, carbamate and H₂O obtained at the end of the medium-pressure decomposition step d) (MPD) is fed, obtaining an off-gas stream (31) comprising NH₃, CO₂ and water, which is fed to the process-water treatment step (PWT), and a fourth stream (32) comprising urea and H₂O;
f) a urea concentration step (CON) to which the fourth stream (32) comprising urea and H₂O obtained at the end of the low-pressure decomposition step e) (LPD) is fed, obtaining a stream of concentrated urea (33) which is fed to the off-gas scrubbing step (OGQ) with molten urea and a stream of H₂O (34) which is fed to the process-water treatment step (PWT).

2. The process according to claim 1, wherein the stream (23), comprising NH₃, CO₂ and melamine vapours, fed to the scrubbing step with urea (OGQ), is only composed of the gaseous stream (21), comprising NH₃, CO₂ and melamine vapours, obtained together with a liquid stream (3) of raw melamine comprising melamine, unreacted urea, oxidized intermediate products of OAT pyrolysis and deammoniation and condensation products of melamine (polycondensates), from the separation of a biphasic liquid-gaseous effluent produced by the urea pyrolysis reaction (R), or said stream (23), comprising NH₃, CO₂ and melamine vapours, fed to the scrubbing step with urea OGQ, comprises said first stream of anhydrous off-gas (21) and a second stream (22) of anhydrous off-gas comprising NH₃, CO₂ and melamine vapour, obtained by putting said liquid stream (3) of raw melamine in contact, in a post-reaction and stripping with ammonia step (PR), with a stream of gaseous NH₃, anhydrous and superheated under pressure (16P), to form a liquid stream (4) of raw melamine depleted of CO₂ and said second stream (22) of anhydrous off-gas.

3. The process according to claim 2, wherein said liquid stream (4) of raw melamine depleted of CO₂, before being subjected to a purification step (P) and a crystallization step (C) by cooling, is subjected to a quench-ammonolysis treatment (QAL) through contact with at least one aqueous ammonia stream (12), with the formation of an aqueous ammonia stream (5) comprising purified melamine substantially free of polycondensates, said quench-ammonolysis treatment (QAL) being preferably carried out by contact with said aqueous ammonia stream (12) coming from the process-water treatment step (PWT) and with a pure ammonia stream (15) coming from the ammonia recovery step (AR).

4. The process according to claim 3, wherein said aqueous ammonia stream (5), comprising purified melamine substantially free of polycondensates, is fed to the purification step (P) from which a solution of purified melamine (6) is obtained, fed to the cold crystallization step (C), from which a suspension of melamine (7) is obtained, then fed to a separation step (S) from which a stream of pure melamine (200) and a stream of mother liquor (9) are obtained, said mother liquor stream (9) comprising H₂O, traces of melamine and OATs being partly fed to the process-water treatment step (PWT), where it is put in contact with the aqueous stream (34) coming from step f) and with the off-gas stream (31) comprising NH₃, CO₂ and water coming from the low-pressure decomposition (LPD) step e), obtaining at the end of the process-water treatment step (PWT) where melamine and OATs are decomposed by hydrolysis at a temperature ranging from 270°C to 300°C, a stream of pure water (300), the liquid stream (12) comprising ammonia and water which is sent to the quench-ammonolysis step (QAL), a liquid stream (13) comprising water, ammonia and CO₂ which is sent to the ammonia recovery step (AR) and the gaseous stream (14) comprising water, ammonia and CO₂ which is sent to the off-gas condensation section (OGC).

5. The process according to one or more of claims 1 to 4, wherein said ammonia recovery step (AR) removes from the liquid stream (13) comprising water, ammonia and CO₂ coming from the process-water treatment step (PWT) and from the stream of off-gas (29) coming from the medium-pressure decomposition step (MPD), at least a part of the ammonia contained therein, forming a liquid stream (17) comprising NH₃, CO₂ and water which is sent to step a) for the condensation of the off-gas (OGC) and a stream of pure ammonia which is sent partly (15) to the quench-ammonolysis step (QAL), partly (16R) to the pyrolysis reaction (R) and partly (16P), to the post-reaction and stripping with ammonia step (PR).

6. The process according to one or more of claims 1 to 5, wherein the urea fed to the off-gas scrubbing step (OGQ) is concentrated urea (33) coming from the urea concentration step f) (CON), possibly with the addition of a stream (100) of molten urea from the battery limits, the two streams (21, 22) of anhydrous off-gas comprising NH₃ CO₂ and melamine vapours coming from the reaction step (R) and from the post-reaction and stripping with ammonia step (PR), being fed, together or separately, to said off-gas scrubbing step (OGQ) with urea, obtaining a stream (24) of scrubbed off-gas which is sent to step a) for the condensation of the off-gas (OGC) and a stream of urea (2) comprising the melamine removed from the off-gas stream (23), which is recycled to the reaction step (R).

7. The process according to one or more of claims 1 to 6, wherein the molten urea fed to the off-gas scrubbing step (OGQ) is only concentrated urea (33) coming from the urea concentration step f) (CON), a stream of liquid ammonia (101) and a stream of gaseous CO₂ (102) being fed to the conversion step (OGR) to be put in contact with the liquid carbamate stream (25) comprising NH₃, CO₂ and H₂O coming from the off-gas condensation (OGC) step a) to obtain the first stream (26) containing urea, ammonia, carbamate and water, in turn sent to the high-pressure decomposition (HPD) step c).

8. The process according to one or more of claims 1 to 7, wherein, in the process-water treatment step (PWT), the mother liquor stream (9), comprising H₂O, traces of melamine and OATs, is subjected to a decomposition treatment by hydrolysis at a temperature ranging from 270°C to 300°C, with the formation of the gaseous stream (14), comprising NH₃, CO₂ and H₂O, and a liquid stream subjected to a stripping treatment in order to obtain a liquid stream of pure water (300).

9. A plant for the production of melamine, comprising:
- a condensation section (OGC), carried out at a pressure ranging from 70 to 220 bar, preferably 80-150 bar, suitable for condensing
i. a gaseous stream of anhydrous off-gas comprising NH₃ and CO₂, coming via line (24) from an off-gas scrubbing section (OGQ) with urea, a stream comprising NH₃, CO₂ and melamine vapours, produced as a gaseous effluent from the urea pyrolysis reaction and coming via a line (23) from the reaction section (R) and from the post-reaction and ammonia stripping section (PR),
ii. a liquid stream comprising NH₃, CO₂ and H₂O, coming via a line (17) from an ammonia recovery section (AR);
iii. a gaseous stream comprising NH₃, CO₂ and H₂O coming via a line (14) from a process water treatment section (PWT) and
iv. a gaseous stream of off-gas comprising NH₃, CO₂ and H₂O, coming via a line (27) from a high-pressure off-gas decomposition section (HPD);
said condensation section (OGC) being connected via supply lines (24, 17, 14, 27) to the scrubbing (OGQ), ammonia recovery (AR), process-water treatment (PWT) and high-pressure decomposition (HPD) sections, from which it receives the above-mentioned streams i.-iv.) at the same pressure present in the condensation section (OGC);
said condensation section (OGC) being connected via an outlet line (25) to a conversion section (OGR) to which it feeds a liquid stream comprising NH₃, CO₂ and H₂O;
- the conversion section (OGR) to which said liquid stream comprising NH₃, CO₂ and H₂O coming from the condensation section (OGC) is fed via line (25), is connected to a high-pressure decomposition section (HPD) to which it feeds via line (26) a first stream comprising urea, ammonia, carbamate and H₂O;
- the high-pressure decomposition section (HPD) to which the first stream comprising urea, ammonia, carbamate and H₂O is fed via line (26), is connected via line (27) to the condensation section (OGC) and via line (28) to a medium-pressure decomposition section (MPD) to which it feeds, via line (28), a second stream comprising urea, carbamate and H₂O;
- the medium-pressure decomposition section (MPD) to which the second stream comprising urea, carbamate and H₂O is fed via line (28), is connected via line (29) to the ammonia recovery section (AR) to which it feeds an off-gas stream comprising NH₃, CO₂ and H₂O, and via line (30) to a low-pressure decomposition section (LPD) to which it feeds a third stream comprising urea, carbamate and H₂O;
- the low-pressure decomposition section (LPD) to which the third stream comprising urea, carbamate and H₂O from the medium-pressure decomposition section (MPD) is fed, via line (30), is connected via a line (31) to the process-water treatment section (PWT) to which an off-gas stream comprising NH₃, CO₂ and H₂O is fed, and via a line (32) to the urea concentration section (CON) to which a fourth stream comprising urea and H₂O is fed;
- the urea concentration section (CON) to which the fourth stream comprising urea and H₂O is fed, via line (32), from the low-pressure decomposition section (LPD), is connected via a line (33) to the off-gas scrubbing section (OGQ) to which it feeds a stream of concentrated urea and via a line (34) to the process-water treatment section (PWT) to which it feeds a stream of H₂O.

10. The plant according to claim 9, wherein the urea scrubbing section (OGQ) is connected via a line (23), to the reaction section (R) and post-reaction and ammonia stripping section (PR) from which it receives, via lines (21, 22), a stream of anhydrous off-gas comprising NH₃, CO₂ and melamine vapours, said reaction section (R) being connected via a line (3) to the post-reaction section (PR) to which it feeds a liquid stream of raw melamine comprising melamine, unreacted urea, oxidized intermediate products of pyrolysis (OATs) and deammoniation and condensation products of melamine (polycondensates), said reaction (R) and post-reaction and stripping sections with ammonia (PR) being respectively connected, via lines (16R, 16P), to the ammonia recovery section (AR) from which they receive a stream of gaseous, anhydrous and superheated ammonia under pressure (16R) and a stream of gaseous ammonia, anhydrous and superheated under pressure (16P), said post-reaction section (PR) being connected via a line (4) to the quench-ammonolysis section (QAL) to which it feeds, via line (4), a liquid stream of raw melamine depleted of CO₂, said quench-ammonolysis section (QAL) being connected, via lines (15, 12 and 10), to the ammonia recovery section (AR) from which it receives a stream of pure ammonia, to the process-water treatment section (PWT) from which it receives an aqueous ammonia solution, to a separation section (S) from which it receives a stream of mother liquor, said quench-ammonolysis section (QAL) being finally connected, via a line (5), to a purification section (P) to which it feeds an aqueous ammonia stream comprising purified melamine substantially free of polycondensates, said purification section (P) being connected via a line (6) to the cold crystallization section (C) to which it feeds a purified melamine solution, said cold crystallization section (C) being connected via a line (7) to a separation section (S) to which a melamine suspension is fed, said separation section (S) being connected via a line (9) to the process-water treatment section (PWT) to which it feeds a first part of the mother liquor comprising H₂O, traces of melamine and OATs to be thermally decomposed at a temperature ranging from 270°C to 300°C, and via a line (10) to the quench-ammonolysis section (QAL) to which it feeds a second part of the mother liquor comprising H₂O, traces of melamine and OATs, and, via a line (200), to the outside of the plant where a stream of pure melamine is sent.

11. The plant according to one or more of claims 9 and 10, wherein the process-water treatment section (PWT) is connected via a line (31), to the low-pressure decomposition section (LPD) from which it receives, via said line (31), an off-gas stream comprising NH₃, CO₂ and H₂O, via a line (34), to the urea concentration section (CON) from which it receives an aqueous stream, and via a line (300), to the outside of the plant where a stream of pure water is sent, said process-water treatment section (PWT) also being connected via a line (12), to the quench-ammonolysis section (QAL) to which it feeds a liquid stream comprising ammonia and water, via a line (13), to the ammonia recovery section (AR) to which it feeds a liquid stream comprising water, ammonia and CO₂ and, via a line (14), to the off-gas condensation section (OGC) to which it feeds a gaseous stream comprising NH₃, CO₂ and H₂O.

12. The plant according to one or more of claims 9-11, wherein the off-gas scrubbing section (OGQ) is connected via a line (33), to the urea concentration section (CON) from which it receives a stream of concentrated urea and, via a line (100), with the outside of the plant from which it receives a stream of molten urea from the battery limits, said off-gas scrubbing section (OGQ) being then connected via a line (2), with the reaction section (R) to which it feeds a urea recycling stream comprising melamine removed from the off-gas stream (23).

13. The plant according to one or more of claims 9-12, wherein the off-gas scrubbing section (OGQ) is connected, via a line (33), to the urea concentration section (CON) from which it receives a stream of concentrated urea and wherein the conversion section (OGR), via lines (101, 102), with the outside of the plant from which it receives a stream of liquid ammonia and a stream of gaseous CO₂.

14. The plant according to one or more of claims 9-13, wherein the urea concentration section (CON) is connected, via a line (110), with the outside of the plant from which it receives a stream of urea solution from the battery limits, and, via a line (33), to the off-gas scrubbing section (OGQ) to which it feeds a stream of concentrated urea, said off-gas scrubbing section (OGQ) being then connected, via a line (2), to the reaction section (R) to which it feeds a urea recycling stream comprising the melamine removed from the off-gas stream (23).

## Patentansprüche

1. Verfahren zur Herstellung von Melamin mittels einer Hochdruck-Harnstoffpyrolysereaktion, umfassend die folgenden Arbeitsschritte:
a) einen Abgaskondensationsschritt (OGC), der bei einem Druck im Bereich von 70 bis 220 bar, vorzugsweise 80-150 bar, durchgeführt wird, dem die folgenden Ströme zugeführt werden
i) einen gasförmigen Strom aus wasserfreiem Abgas (24), der NH₃ und CO₂ umfasst und aus einem Waschschritt des Abgases (OGQ) mit Harnstoff kommt, einen Strom (23), der NH₃, CO₂ und Melamindämpfe umfasst, die als gasförmiges Abwasser aus der Harnstoffpyrolysereaktion in dem Reaktionsteil (R) und dem Nachreaktions- und Ammoniakstrippteil (PR) des Melaminsyntheseverfahrens erzeugt werden,
ii) einen flüssigen Strom (17), der NH₃, CO₂ und H₂O umfasst und aus einem Ammoniakrückgewinnungsschritt (AR) kommt;
iii) einen gasförmigen Strom (14), der NH₃, CO₂ und H₂O umfasst und aus einem Prozesswasseraufbereitungsschritt (PWT) kommt und
iv) einen gasförmigen Abgasstrom (27), der NH₃, CO₂ und H₂O umfasst und aus einem Hochdruckabgaszersetzungsschritt (HPD) kommt;
wobei die Ströme i.-iv. mit dem gleichen Druck, bei dem der Kondensationsschritt (OGC) durchgeführt wird, dem Abgaskondensationsschritt (OGC) zugeführt werden und einen flüssigen Strom (25) erhalten wird, der NH₃, CO₂ und H₂O umfasst;
b) einen Umwandlungsschritt (OGR), dem der am Ende des Schritts a) erhaltene, NH₃, CO₂ und H₂O umfassende flüssige Strom (25) zugeführt wird, wobei ein erster Strom (26) erhalten wird, der Harnstoff, Ammoniak, Carbamat und H₂O umfasst;
c) einen Hochdruckzersetzungsschritt (HPD), dem der am Ende des Umwandlungsschritts b) erhaltene, Harnstoff, Ammoniak, Carbamat und H₂O umfassende erste Strom (26) zugeführt wird, wobei der dem Kondensationsschritt a) zugeführte Strom iv) (27) und ein Harnstoff, Carbamat und H₂O umfassender zweiter Strom (28) erhalten werden;
d) einen Mitteldruckzersetzungsschritt (MPD), dem der am Ende des Hochdruckzersetzungsschritts c) (HPD) erhaltene, Harnstoff, Carbamat und H₂O umfassende zweite Strom (28) zugeführt wird, wobei ein NH₃, CO₂ und Wasser umfassender Abgasstrom (29), der dem Ammoniakrückgewinnungsschritt (AR) zugeführt wird, und ein Harnstoff, Carbamat und H₂O umfassender dritter Strom (30) erhalten werden;
e) einen Niederdruckzersetzungsschritt (LPD), dem der am Ende des Mitteldruckzersetzungsschritts d) (MPD) erhaltene, Harnstoff, Carbamat und H₂O umfassende dritte Strom (30) zugeführt wird, wobei ein NH₃, CO₂ und Wasser umfassender Abgasstrom (31), der dem Prozesswasseraufbereitungsschritt (PWT) zugeführt wird, und ein Harnstoff und H₂O umfassender vierter Strom (32) erhalten werden;
f) einen Harnstoffkonzentrierungsschritt (CON), dem der am Ende des Niederdruckzersetzungsschritts e) (LPD) erhaltene, Harnstoff und H₂O umfassende vierte Strom (32) zugeführt wird, wobei ein Strom aus konzentriertem Harnstoff (33), der dem Abgaswaschschritt (OGQ) mit geschmolzenem Harnstoff zugeführt wird, und ein Strom aus H₂O (34), der dem Prozesswasseraufbereitungsschritt (PWT) zugeführt wird, erhalten werden.

2. Verfahren nach Anspruch 1, wobei der dem Waschschritt mit Harnstoff (OGQ) zugeführte, NH₃, CO₂ und Melamindämpfe umfassende Strom (23) nur aus dem NH₃, CO₂ und Melamindämpfe umfassenden gasförmigen Strom (21) zusammengesetzt ist, erhalten zusammen mit einem flüssigen Strom (3) von Rohmelamin, der Melamin, nicht umgesetzten Harnstoff, oxidierte Zwischenprodukte der OAT-Pyrolyse und Deammonisierungs- und Kondensationsprodukte von Melamin (Polykondensate) umfasst, aus der Abtrennung eines zweiphasigen, flüssigen-gasförmigen Abwassers, das durch die Harnstoffpyrolysereaktion (R) erzeugt wird, oder der Strom (23), der NH₃, CO₂ und Melamindämpfe umfasst, dem Waschschritt mit Harnstoff-OGQ zugeführt wird, den ersten Strom aus wasserfreiem Abgas (21) und einen zweiten Strom (22) aus wasserfreiem Abgas umfasst, der NH₃, CO₂ und Melamindämpfe umfasst, die erhalten werden, indem der flüssige Strom (3) von Rohmelamin, in einer Nachreaktion und Stripping mit einem Ammoniakschritt (PR), in Kontakt mit einem Strom von gasförmigem NH₃, wasserfrei und unter Druck (16P) überhitzt, gebracht wird, um einen flüssigen Strom (4) von Rohmelamin, das an CO₂ abgereichert ist, und den zweiten Strom (22) von wasserfreiem Abgas zu bilden.

3. Verfahren nach Anspruch 2, wobei der flüssige Strom (4) von an CO₂ abgereichertem Rohmelamin, bevor er einem Reinigungsschritt (P) und einem Kristallisationsschritt (C) durch Abkühlen unterzogen wird, einer Quench-Ammonolysebehandlung (QAL) durch Kontakt mit mindestens einem wässrigen Ammoniakstrom (12) unterzogen wird, wobei ein wässriger Ammoniakstrom (5) gebildet wird, der gereinigtes Melamin umfasst, das im Wesentlichen frei von Polykondensaten ist, wobei die Quench-Ammonolysebehandlung (QAL) vorzugsweise durch Kontakt mit dem wässrigen Ammoniakstrom (12), der aus dem Prozesswasseraufbereitungsschritt (PWT) kommt, und mit einem reinen Ammoniakstrom (15), der aus dem Ammoniakrückgewinnungsschritt (AR) kommt, durchgeführt wird.

4. Verfahren nach Anspruch 3, wobei der wässrige Ammoniakstrom (5), der gereinigtes Melamin umfasst, das im Wesentlichen frei von Polykondensaten ist, dem Reinigungsschritt (P) zugeführt wird, aus dem eine Lösung von gereinigtem Melamin (6) erhalten wird, dem Kaltkristallisationsschritt (C) zugeführt wird, aus dem eine Suspension von Melamin (7) erhalten wird, dann einem Trennschritt (S) zugeführt wird, aus dem ein Strom von reinem Melamin (200) und ein Strom von Mutterlauge (9) erhalten werden, wobei der Mutterlaugenstrom (9), der H₂O, Spuren von Melamin und OATs umfasst, teilweise dem Prozesswasseraufbereitungsschritt (PWT) zugeführt werden, in dem er mit dem wässrigen Strom (34) aus Schritt f) und mit dem Abgasstrom (31), der NH₃, CO₂ und Wasser umfasst und aus dem Niederdruckzersetzungsschritt (LPD) Schritt e) kommt, in Kontakt gebracht wird, wobei am Ende des Prozesswasseraufbereitungsschritts (PWT), in dem Melamin und OATs durch Hydrolyse bei einer Temperatur im Bereich von 270 °C bis 300 °C zersetzt werden, ein Strom aus reinem Wasser (300), der flüssige Strom (12), der Ammoniak und Wasser umfasst, der dem Quench-Ammonolyseschritt (QAL) geleitet wird, ein flüssiger Strom (13), der Wasser, Ammoniak und CO₂ umfasst, der dem Ammoniakrückgewinnungsschritt (AR) geleitet wird, und der gasförmige Strom (14), der Wasser, Ammoniak und CO₂ umfasst, der dem Abgaskondensationsteil (OGC) geleitet wird, erhalten werden.

5. Verfahren nach einem oder mehreren der Ansprüche 1 bis 4, wobei der Ammoniakrückgewinnungsschritt (AR) aus dem flüssigen Strom (13), der Wasser, Ammoniak und CO₂ umfasst und aus dem Prozesswasseraufbereitungsschritt (PWT) kommt, und aus dem Abgasstrom (29), der aus dem Mitteldruckzersetzungsschritt (MPD) kommt, mindestens einen Anteil des darin enthaltenen Ammoniaks entfernt, wobei ein flüssiger Strom (17), der NH₃, CO₂ und Wasser, der dem Schritt a) zur Kondensation des Abgases (OGC) geleitet wird, und ein Strom aus reinem Ammoniak, der teilweise (15) dem Quench-Ammonolyseschritt (QAL), teilweise (16R) der Pyrolysereaktion (R) und teilweise (16P), der Nachreaktion und Stripping mit Ammoniak-Schritt (PR) geleitet wird, gebildet werden.

6. Verfahren nach einem oder mehreren der Ansprüche 1 bis 5, wobei der dem Abgaswaschschritt (OGQ) zugeführte Harnstoff konzentrierter Harnstoff (33) ist, der aus dem Harnstoffkonzentrierungsschritt f) (CON), gegebenenfalls unter Zugabe eines Stroms (100) von geschmolzenem Harnstoff aus den integrierten Anlagen, kommt, wobei die zwei Ströme (21, 22) von wasserfreiem Abgas, das NH₃ CO₂ und Melamindämpfe umfasst, die aus dem Reaktionsschritt (R) und aus der Nachreaktion und Strippen mit Ammoniakschritt (PR) kommen, gemeinsam oder getrennt dem Abgaswaschschritt (OGQ) mit Harnstoff zugeführt werden, wobei ein Strom (24) von gewaschenem Abgas, der zu Schritt a) zur Kondensation des Abgases (OGC) geleitet wird, und ein Harnstoffstrom (2) erhalten werden, der das aus dem Abgasstrom (23) entfernte Melamin umfasst, das in den Reaktionsschritt (R) zurückgeführt wird.

7. Verfahren nach einem oder mehreren der Ansprüche 1 bis 6, wobei der geschmolzene Harnstoff, der dem Abgaswaschschritt (OGQ) zugeführt wird, nur konzentrierter Harnstoff (33) ist, der aus dem Harnstoffkonzentrierungsschritt f) (CON) kommt, wobei ein Strom von flüssigem Ammoniak (101) und ein Strom von gasförmigem CO₂ (102) dem Umwandlungsschritt (OGR) zugeführt werden, um mit dem flüssigen Carbamatstrom (25) in Kontakt gebracht zu werden, der NH₃, CO₂ und H₂O umfasst und aus dem Abgaskondensationsschritt (OGC) a) kommt, um den ersten Strom (26) zu erhalten, der Harnstoff, Ammoniak, Carbamat und Wasser enthält, und der wiederum dem Hochdruckzersetzungsschritt (HPD) c) geleitet wird.

8. Verfahren nach einem oder mehreren der Ansprüche 1 bis 7, wobei im Prozesswasseraufbereitungsschritt (PWT) der Mutterlaugenstrom (9), der H₂O, Spuren von Melamin und OATs umfasst, einer Zersetzungsbehandlung durch Hydrolyse bei einer Temperatur im Bereich von 270 °C bis 300 °C unterzogen wird, mit Bildung des gasförmigen Stroms (14), umfassend NH₃, CO₂ und H₂O, und eines flüssigen Stroms, der einer Strippbehandlung unterzogen wird, um einen flüssigen Strom aus reinem Wasser (300) zu erhalten.

9. Anlage zur Herstellung von Melamin, umfassend:
- einen Kondensationsteil (OGC), der bei einem Druck im Bereich von 70 bis 220 bar, vorzugsweise 80-150 bar, durchgeführt wird und zum Kondensieren geeignet ist
i. eines gasförmigen Stroms wasserfreien Abgases, der NH₃ und CO₂ umfasst und über Leitung (24) aus einem Abgaswaschteil (OGQ) mit Harnstoff kommt, eines Stroms, der NH₃, CO₂ und Melamindämpfe umfasst, der als gasförmiges Abwasser aus der Harnstoffpyrolysereaktion erzeugt und über eine Leitung (23) aus dem Reaktionsteil (R) und aus dem Nachreaktions- und Ammoniakstrippteil (PR) kommt,
ii. eines flüssigen Stroms, der NH₃, CO₂ und H₂O umfasst und über eine Leitung (17) aus einem Ammoniakrückgewinnungsteil (AR) kommt;
iii. eines gasförmigen Stroms, der NH₃, CO₂ und H₂O umfasst und über eine Leitung (14) aus einem Prozesswasseraufbereitungsteil (PWT) kommt und
iv. eines gasförmigen Abgasstroms, der NH₃, CO₂ und H₂O umfasst und über eine Leitung (27) aus einem Hochdruckabgaszersetzungsteil (HPD) kommt;
wobei der Kondensationsteil (OGC) über Versorgungsleitungen (24, 17, 14, 27) mit den Wasch- (OGQ), Ammoniakrückgewinnungs- (AR), Prozesswasseraufbereitungs- (PWT) und Hochdruckzersetzungs- (HPD)-Teilen verbunden ist, aus denen er die oben genannten Ströme i-iv.) mit dem gleichen Druck empfängt, der im Kondensationsteil (OGC) vorhanden ist; wobei der Kondensationsteil (OGC) über eine Auslassleitung (25) mit einem Umwandlungsteil (OGR) verbunden ist, dem er einen flüssigen Strom zuführt, der NH₃, CO₂ und H₂O umfasst;
- der Umwandlungsteil (OGR), dem der aus dem Kondensationsteil (OGC) kommende und NH₃, CO₂ und H₂O umfassende flüssige Strom über Leitung (25) zugeführt wird, mit einem Hochdruckzersetzungsteil (HPD) verbunden ist, dem er über Leitung (26) einen Harnstoff, Ammoniak, Carbamat und H₂O umfassenden ersten Strom zuführt;
- der Hochdruckzersetzungsteil (HPD), dem der Harnstoff, Ammoniak, Carbamat und H₂O umfassende erste Strom über Leitung (26) zugeführt wird, über Leitung (27) mit dem Kondensationsteil (OGC) und über Leitung (28) mit einem Mitteldruckzersetzungsteil (MPD) verbunden ist, dem er über Leitung (28) einen Harnstoff, Carbamat und H₂O umfassenden zweiten Strom zuführt;
- der Mitteldruckzersetzungsteil (MPD), dem der Harnstoff, Carbamat und H₂O umfassende zweite Strom über Leitung (28) zugeführt wird, über Leitung (29) mit dem Ammoniakrückgewinnungsteil (AR), dem er einen NH₃, CO₂ und H₂O umfassenden Abgasstrom zuführt, und über Leitung (30) mit einem Niederdruckzersetzungsteil (LPD), dem er einen Harnstoff, Carbamat und H₂O umfassenden dritten Strom zuführt, verbunden ist;
- der Niederdruckzersetzungsteil (LPD), dem der Harnstoff, Carbamat und H₂O umfassende dritte Strom aus dem Mitteldruckzersetzungsteil (MPD) über Leitung (30) zugeführt wird, über Leitung (31) mit dem Prozesswasseraufbereitungsteil (PWT), dem ein NH₃, CO₂ und H₂O umfassender Abgasstrom zugeführt wird, und über Leitung (32) mit dem Harnstoffkonzentrationsteil (CON), dem ein Harnstoff und H₂O umfassender vierter Strom zugeführt wird, verbunden ist;
- der Harnstoffkonzentrationsteil (CON), dem der Harnstoff und H₂O umfassende vierte Strom über Leitung (32) aus dem Niederdruckzersetzungsteil (LPD) zugeführt wird, über Leitung (33) mit dem Abgaswaschteil (OGQ), dem er einen Strom aus konzentriertem Harnstoff zuführt, und über Leitung (34) mit dem Prozesswasseraufbereitungsteil (PWT), dem er einen Strom aus H₂O zuführt, verbunden ist.

10. Anlage nach Anspruch 9, wobei der Harnstoffwaschteil (OGQ) über eine Leitung (23) mit dem Reaktionsteil (R) und dem Nachreaktions- und Ammoniakstrippteil (PR) verbunden ist, aus dem er über Leitungen (21, 22) einen wasserfreien Abgasstrom, der NH₃, CO₂ und Melamindämpfe umfasst, empfängt, wobei der Reaktionsteil (R) über eine Leitung (3) mit dem Nachreaktionsteil (PR) verbunden ist, dem er einen flüssigen Strom von Rohmelamin, der Melamin, nicht umgesetzten Harnstoff, oxidierte Zwischenprodukte der Pyrolyse (OATs) und Deammonisierungs- und Kondensationsprodukte von Melamin (Polykondensate) umfasst, zuführt, wobei die Reaktions- (R) und Nachreaktions- und Strippteile mit Ammoniak (PR) jeweils über Leitungen (16R, 16P) mit dem Ammoniakrückgewinnungsteil (AR) verbunden sind, aus dem sie einen Strom von gasförmigem, wasserfreiem und überhitztem Ammoniak unter Druck (16R) und einen Strom von gasförmigem Ammoniak, wasserfrei und unter Druck (16P) überhitzt, empfangen, wobei der Nachreaktionsteil (PR) über eine Leitung (4) mit dem Quench-Ammonolyseteil (QAL) verbunden ist, dem er über Leitung (4), einen flüssigen Strom von an CO₂ abgereichertem Rohmelamin zuführt, wobei der Quench-Ammonolyseteil (QAL) über Leitungen (15, 12 und 10) mit dem Ammoniakrückgewinnungsteil (AR), aus dem er einen Strom von reinem Ammoniak empfängt, mit dem Prozesswasseraufbereitungsteil (PWT), aus dem er eine wässrige Ammoniaklösung empfängt, mit einem Trennteil (S), aus dem er einen Strom von Mutterlauge empfängt, verbunden ist, wobei der Quench-Ammonolyseteil (QAL) schließlich über eine Leitung (5) mit einem Reinigungsteil (P) verbunden ist, dem er einen wässrigen Ammoniakstrom zuführt, der gereinigtes Melamin umfasst, das im Wesentlichen frei von Polykondensaten ist, wobei der Reinigungsteil (P) über eine Leitung (6) mit dem Kaltkristallisationsteil (C) verbunden ist, dem er eine gereinigte Melaminlösung zuführt, wobei der Kaltkristallisationsteil (C) über eine Leitung (7) mit einem Trennteil (S) verbunden ist, dem eine Melaminsuspension zugeführt wird, wobei der Trennteil (S) über eine Leitung (9) mit dem Prozesswasseraufbereitungsteil (PWT), dem er einen ersten Anteil an Mutterlauge, die H₂O, Spuren von Melamin und OATs umfasst, zuführt, die bei einer Temperatur im Bereich von 270 °C bis 300 °C thermisch zersetzt werden sollen, und über eine Leitung (10) mit dem Quench-Ammonolyseteil (QAL), dem er einen zweiten Anteil an Mutterlauge, die H₂O, Spuren von Melamin und OATs umfasst, zuführt, und über eine Leitung (200) zur Außenseite der Anlage, wo ein Strom von reinem Melamin geleitet wird, verbunden ist.

11. Anlage nach einem oder mehreren der Ansprüche 9 und 10, wobei der Prozesswasseraufbereitungsteil (PWT) über eine Leitung (31) mit dem Niederdruckzersetzungsteil (LPD), aus dem er über die Leitung (31) einen NH₃, CO₂ und H₂O umfassenden Abgasstrom empfängt, über eine Leitung (34) mit dem Harnstoffkonzentrierungsteil (CON), aus dem er einen wässrigen Strom empfängt, und über eine Leitung (300), zur Außenseite der Anlage, wo ein Strom von reinem Wasser geleitet wird, verbunden ist, wobei der Prozesswasseraufbereitungsteil (PWT) auch über eine Leitung (12) mit dem Quench-Ammonolyseteil (QAL), dem er über eine Leitung (13) einen Ammoniak und Wasser umfassenden flüssigen Strom zuführt, mit dem Ammoniakrückgewinnungsteil (AR), dem er einen Wasser, Ammoniak und CO₂ umfassenden flüssigen Strom zuführt und über eine Leitung (14) mit dem Abgaskondensationsteil (OGC), dem er einen NH₃, CO₂ und H₂O umfassenden gasförmigen Strom zuführt, verbunden ist.

12. Anlage nach einem oder mehreren der Ansprüche 9-11, wobei der Abgaswaschteil (OGQ) über eine Leitung (33) mit dem Harnstoffkonzentrierungsteil (CON), aus dem er einen Strom von konzentriertem Harnstoff empfängt und über eine Leitung (100) mit der Außenseite der Anlage, von der er einen Strom von geschmolzenem Harnstoff von den integrierten Anlagen empfängt, verbunden ist, wobei der Abgaswaschteil (OGQ) dann über eine Leitung (2) mit dem Reaktionsteil (R) verbunden ist, dem er einen Harnstoffrecyclingstrom zuführt, der Melamin umfasst, das aus dem Abgasstrom (23) entfernt wurde.

13. Anlage nach einem oder mehreren der Ansprüche 9-12, wobei der Abgaswaschteil (OGQ) über eine Leitung (33) mit dem Harnstoffkonzentrierungsteil (CON) verbunden ist, aus dem er einen Strom von konzentriertem Harnstoff empfängt, und wobei der Umwandlungsteil (OGR) über Leitungen (101, 102) mit der Außenseite der Anlage verbunden ist, aus der er einen Strom von flüssigem Ammoniak und einen Strom von gasförmigem CO₂ empfängt.

14. Anlage nach einem oder mehreren der Ansprüche 9-13, wobei der Harnstoffkonzentrationsteil (CON) über eine Leitung (110) mit der Außenseite der Anlage, aus der er einen Strom von Harnstofflösung von den integrierten Anlagen empfängt, und über eine Leitung (33) mit dem Abgaswaschteil (OGQ) verbunden ist, dem er einen Strom von konzentriertem Harnstoff zuführt, wobei der Abgaswaschteil (OGQ) dann über eine Leitung (2) mit dem Reaktionsteil (R) verbunden ist, dem er einen Harnstoffrecyclingstrom zuführt, der Melamin umfasst, das aus dem Abgasstrom (23) entfernt wurde.

## Revendications

1. Procédé de production de mélamine au moyen d'une réaction de pyrolyse d'urée à haute pression, comprenant les étapes suivantes :
a) une étape de condensation de gaz résiduel (OGC), réalisée à une pression comprise entre 70 et 220 bars, de préférence entre 80 et 150 bars, dans laquelle sont introduits les flux suivants
i) un flux gazeux de gaz résiduel anhydre (24) comprenant du NH₃ et du CO₂, provenant d'une étape de lavage de gaz résiduel (OGQ) avec de l'urée, un flux (23) comprenant du NH₃, du CO₂ et des vapeurs de mélamine, produit en tant qu'effluent gazeux de la réaction de pyrolyse de l'urée dans la section de réaction (R) et la section de post-réaction et de décapage à l'ammoniac (PR) du procédé de synthèse de la mélamine,
ii) un flux liquide (17) comprenant du NH₃, du CO₂ et de l'H₂O, provenant d'une étape de récupération de l'ammoniac (AR) ;
iii) un flux gazeux (14) comprenant du NH₃, du CO₂ et de l'H₂O provenant d'une étape de traitement de l'eau de traitement (PWT) et
iv) un flux gazeux de gaz résiduel (27) comprenant du NH₃, du CO₂ et de l'H₂O, provenant d'une étape de décomposition de gaz résiduel à haute pression (HPD) ;
lesdits flux i.-iv. sont introduits dans l'étape de condensation de gaz résiduel (OGC) à la même pression que celle à laquelle l'étape de condensation (OGC) est effectuée, et qui permet d'obtenir un flux liquide (25) comprenant du NH₃, du CO₂ et de l'H₂O ;
b) une étape de conversion (OGR) dans laquelle ledit flux liquide (25) comprenant du NH₃, du CO₂ et de l'H₂O obtenu à la fin de ladite étape a) est introduit, ce qui permet d'obtenir un premier flux (26) comprenant de l'urée, de l'ammoniac, du carbamate et de l'H₂O ;
c) une étape de décomposition à haute pression (HPD) dans laquelle le premier flux (26) comprenant de l'urée, de l'ammoniac, du carbamate et de l'H₂O obtenu à la fin de l'étape de conversion b) est introduit, ce qui permet d'obtenir le flux iv) (27) introduit dans l'étape a) de condensation et un deuxième flux (28) comprenant de l'urée, du carbamate et de l'H₂O ;
d) une étape de décomposition à moyenne pression (MPD) dans laquelle le deuxième flux (28) comprenant de l'urée, du carbamate et de l'H₂O obtenu à la fin de l'étape c) de décomposition à haute pression (HPD) est introduit, ce qui permet d'obtenir un flux de gaz résiduel (29) comprenant du NH₃, du CO₂ et de l'eau qui est introduit dans l'étape de récupération de l'ammoniac (AR), ainsi qu'un troisième flux (30) comprenant de l'urée, du carbamate et de l'H₂O ;
e) une étape de décomposition à basse pression (LPD) dans laquelle le troisième flux (30) comprenant de l'urée, du carbamate et de l'H₂O obtenu à la fin de l'étape d) de décomposition à moyenne pression (MPD) est introduit, ce qui permet d'obtenir un flux de gaz résiduel (31) comprenant du NH₃, du CO₂ et de l'eau, qui est introduit dans l'étape de traitement de l'eau de traitement (PWT), ainsi qu'un quatrième flux (32) comprenant de l'urée et de l'H₂O ;
f) une étape de concentration de l'urée (CON) dans laquelle le quatrième flux (32) comprenant l'urée et l'H₂O obtenues à la fin de l'étape e) de décomposition à basse pression (LPD) est introduit, ce qui permet d'obtenir un flux d'urée concentrée (33) qui est introduit dans l'étape de lavage de gaz résiduel (OGQ) avec de l'urée fondue et un flux d'H₂O (34) qui est introduit dans l'étape de traitement de l'eau de traitement (PWT).

2. Procédé selon la revendication 1, dans lequel le flux (23), comprenant du NH₃, du CO₂ et des vapeurs de mélamine, introduit dans l'étape de lavage à l'urée (OGQ), est uniquement composé du flux gazeux (21), comprenant du NH₃, du CO₂ et des vapeurs de mélamine, obtenu en même temps qu'un flux liquide (3) de mélamine brute comprenant de la mélamine, de l'urée n'ayant pas réagi, des produits intermédiaires oxydés de la pyrolyse de l'OAT et des produits de désammoniation et de condensation de la mélamine (polycondensats), provenant de la séparation d'un effluent liquide-gazeux biphasique produit par la réaction de pyrolyse de l'urée (R), soit ledit flux (23), comprenant du NH₃, du CO₂ et des vapeurs de mélamine, introduits dans l'étape de lavage à l'urée OGQ, comprend ledit premier flux de gaz résiduel anhydre (21) et un deuxième flux (22) de gaz résiduel anhydre comprenant du NH₃, du CO₂ et des vapeurs de mélamine, obtenu par la mise en contact dudit flux liquide (3) de mélamine brute, dans une étape de post-réaction et de décapage à l'ammoniac (PR), avec un flux de NH₃ gazeux, anhydre et surchauffé sous pression (16P), pour former un flux liquide (4) de mélamine brute appauvrie en CO₂ et ledit second flux (22) de gaz résiduel anhydre.

3. Procédé selon la revendication 2, dans lequel ledit flux liquide (4) de mélamine brute appauvrie en CO₂, avant d'être soumis à une étape de purification (P) et à une étape de cristallisation (C) par refroidissement, est soumis à un traitement de trempe-ammonolyse (QAL) par contact avec au moins un flux aqueux d'ammoniac (12), avec la formation d'un flux d'ammoniac aqueux (5) comprenant de la mélamine purifiée substantiellement exempte de polycondensats, ledit traitement de trempe-ammonolyse (QAL) étant de préférence effectué par contact avec ledit flux d'ammoniac aqueux (12) provenant de l'étape de traitement de l'eau de traitement (PWT) et avec un flux d'ammoniac pur (15) provenant de l'étape de récupération de l'ammoniac (AR).

4. Procédé selon la revendication 3, dans lequel ledit flux aqueux d'ammoniac (5), comprenant de la mélamine purifiée substantiellement exempte de polycondensats, est introduit dans l'étape de purification (P) à partir de laquelle une solution de mélamine purifiée (6) est obtenue, introduite dans l'étape de cristallisation à froid (C), à partir de laquelle une suspension de mélamine (7) est obtenue, puis introduite dans une étape de séparation (S) à partir de laquelle un flux de mélamine pure (200) et un flux de liqueur mère (9) sont obtenus, ledit flux de liqueur mère (9) comprenant de l'H₂O, des traces de mélamine et des OAT étant partiellement introduits dans l'étape de traitement de l'eau de traitement (PWT), où il est mis en contact avec le flux aqueux (34) provenant de l'étape f) et avec le flux de gaz résiduel (31) comprenant du NH₃, du CO₂ et de l'eau provenant de l'étape e) de décomposition à basse pression (LPD), obtenant à la fin de l'étape de traitement de l'eau de traitement (PWT) où la mélamine et les OAT sont décomposées par hydrolyse à une température comprise entre 270°C et 300°C, un flux d'eau pure (300), le flux liquide (12) comprenant de l'ammoniac et de l'eau qui est envoyé à l'étape de trempe-ammonolyse (QAL), un flux liquide (13) comprenant de l'eau, de l'ammoniac et du CO₂ qui est envoyé à l'étape de récupération de l'ammoniac (AR) et le flux gazeux (14) comprenant de l'eau, de l'ammoniac et du CO₂ qui est envoyé à la section de condensation de gaz résiduel (OGC).

5. Procédé selon l'une ou plusieurs des revendications 1 à 4, dans lequel ladite étape de récupération de l'ammoniac (AR) élimine, du flux liquide (13) comprenant de l'eau, de l'ammoniac et du CO₂ provenant de l'étape de traitement de l'eau de traitement (PWT) et du flux de gaz résiduel (29) provenant de l'étape de décomposition à moyenne pression (MPD), au moins une partie de l'ammoniac qu'il contient, formant un flux liquide (17) comprenant du NH₃, du CO₂ et de l'eau qui est envoyé à l'étape a) pour la condensation du gaz résiduel (OGC) et un flux d'ammoniac pur qui est envoyé en partie (15) à l'étape de trempe-ammonolyse (QAL), en partie (16R) à la réaction de pyrolyse (R) et en partie (16P), à l'étape de post-réaction et de décapage à l'ammoniac (PR).

6. Procédé selon l'une ou plusieurs des revendications 1 à 5, dans lequel l'urée introduite dans l'étape de lavage de gaz résiduel (OGQ) est de l'urée concentrée (33) provenant de l'étape f) de concentration de l'urée (CON), éventuellement additionnée d'un flux (100) d'urée fondue provenant des limites de la batterie, les deux flux (21, 22) de gaz résiduel anhydre comprenant du NH₃ CO₂ et des vapeurs de mélamine provenant de l'étape de réaction (R) et de l'étape de post-réaction et de décapage à l'ammoniac (PR), sont introduits, ensemble ou séparément, dans ladite étape de lavage de gaz résiduel (OGQ) avec de l'urée, ce qui permet d'obtenir un flux (24) de gaz résiduel lavé qui est envoyé à l'étape a) pour la condensation de gaz résiduel (OGC) et un flux d'urée (2) comprenant la mélamine retirée du flux de gaz résiduel (23), qui est recyclé dans l'étape de réaction (R).

7. Procédé selon l'une ou plusieurs des revendications 1 à 6, dans lequel l'urée fondue introduite dans l'étape de lavage de gaz résiduel (OGQ) est uniquement de l'urée concentrée (33) provenant de l'étape f) de concentration de l'urée (CON), un flux d'ammoniac liquide (101) et un flux de CO₂ gazeux (102) étant introduits dans l'étape de conversion (OGR) pour être mis en contact avec le flux de carbamate liquide (25) comprenant du NH₃, du CO₂ et de l'H₂O provenant de l'étape a) de condensation de gaz résiduel (OGC) pour obtenir un premier flux (26) contenant de l'urée, de l'ammoniac, du carbamate et de l'eau, à son tour envoyé à l'étape c) de décomposition à haute pression (HPD).

8. Procédé selon l'une ou plusieurs des revendications 1 à 7, dans lequel, dans l'étape de traitement de l'eau de traitement (PWT), le flux de liqueur mère (9), comprenant de l'H₂O, des traces de mélamine et d'OAT, est soumis à un traitement de décomposition par hydrolyse à une température comprise entre 270°C et 300°C, avec formation du flux gazeux (14), comprenant du NH₃, du CO₂ et de l'H₂O, et d'un flux liquide soumis à un traitement de décapage afin d'obtenir un flux liquide d'eau pure (300).

9. Installation pour la production de mélamine, comprenant :
- une section de condensation (OGC), réalisée à une pression comprise entre 70 et 220 bars, de préférence entre 80 et 150 bars, adaptée à la condensation
i. un flux gazeux de gaz résiduel anhydre comprenant du NH₃ et du CO₂, provenant de la conduite (24) d'une section de décapage de gaz résiduel (OGQ) avec de l'urée, un flux comprenant du NH₃, du CO₂ et des vapeurs de mélamine, produit comme effluent gazeux de la réaction de pyrolyse de l'urée et provenant d'une conduite (23) de la section de réaction (R) et de la section de post-réaction et de décapage à l'ammoniac (PR),
ii. un flux liquide comprenant du NH₃, du CO₂ et de l' H₂O, provenant d'une conduite (17) d'une section de récupération de l'ammoniac (AR) ;
iii. un flux gazeux comprenant du NH₃, du CO₂ et de l'H₂O provenant d'une conduite (14) d'une section de traitement de l'eau de traitement (PWT) et
iv. un flux gazeux de gaz résiduel comprenant du NH₃, du CO₂ et de l'H₂O, provenant d'une conduite (27), d'une section de décomposition de gaz résiduel à haute pression (HPD) ;
ladite section de condensation (OGC) étant reliée par des conduites d'alimentation (24, 17, 14, 27) aux sections de lavage (OGQ), de récupération de l'ammoniac (AR), de traitement de l'eau de traitement (PWT) et de décomposition à haute pression (HPD), dont elle reçoit les flux i.-iv.) susmentionnés à la même pression que celle présente dans la section de condensation (OGC) ;
ladite section de condensation (OGC) étant reliée par une conduite de sortie (25) à une section de conversion (OGR) dans laquelle elle introduit un flux liquide comprenant du NH₃, du CO₂ et de l'H₂O ;
- la section de conversion (OGR) dans laquelle ledit flux liquide comprenant du NH₃, du CO₂ et de l'H₂O provenant de la section de condensation (OGC) est introduit par la conduite (25), est reliée à une section de décomposition à haute pression (HPD) dans laquelle elle introduit par la conduite (26) un premier flux comprenant de l'urée, de l'ammoniac, du carbamate et de l'H₂O ;
- la section de décomposition à haute pression (HPD), dans laquelle le premier flux comprenant de l'urée, de l'ammoniac, du carbamate et de l'H₂O est introduit par la conduite (26), est reliée par la conduite (27) à la section de condensation (OGC) et par la conduite (28) à une section de décomposition à moyenne pression (MPD) dans laquelle elle introduit, par la conduite (28), un deuxième flux comprenant de l'urée, du carbamate et de l'H₂O ;
- la section de décomposition à moyenne pression (MPD), dans laquelle le deuxième flux comprenant de l'urée, du carbamate et de l'H₂O est introduit par la conduite (28), est reliée par la conduite (29) à la section de récupération de l'ammoniac (AR), dans laquelle elle introduit un flux de gaz résiduel comprenant du NH₃, du CO₂ et de l'H₂O, et par la conduite (30) à une section de décomposition à basse pression (LPD), dans laquelle elle introduit un troisième flux comprenant de l'urée, du carbamate et de l'H₂O ;
- la section de décomposition à basse pression (LPD), dans laquelle le troisième flux comprenant de l'urée, du carbamate et de l'H₂O provenant de la section de décomposition à moyenne pression (MPD) est introduit, par la conduite (30), est reliée par une conduite (31), à la section de traitement de l'eau de traitement (PWT), dans laquelle un flux de gaz résiduel comprenant du NH₃, du CO₂ et de l'H₂O est introduit, et à la section de concentration de l'urée (CON), dans laquelle un quatrième flux comprenant de l'urée et de l'H₂O est introduit, par l'intermédiaire d'une conduite (32) ;
- la section de concentration de l'urée (CON), dans laquelle le quatrième flux comprenant de l'urée et de l'H₂O est introduit par la conduite (32) provenant de la section de décomposition à basse pression (LPD), est reliée par une conduite (33) à la section de lavage de gaz résiduel (OGQ), dans laquelle elle introduit un flux d'urée concentrée, et par une conduite (34) à la section de traitement de l'eau de traitement (PWT), dans laquelle elle introduit un flux d'H₂O.

10. Installation selon la revendication 9, dans laquelle la section de lavage de l'urée (OGQ) est reliée par une conduite (23) à la section de réaction (R) et à la section de post-réaction et de décapage à l'ammoniac (PR) dont elle reçoit, par les conduites (21, 22), un flux de gaz résiduel anhydre comprenant du NH₃, du CO₂ et des vapeurs de mélamine, ladite section de réaction (R) est reliée par une conduite (3) à la section de post-réaction (PR) dans laquelle elle introduit un flux liquide de mélamine brute comprenant de la mélamine, de l'urée n'ayant pas réagi, des produits intermédiaires oxydés de la pyrolyse (OAT) et des produits de désammoniation et de condensation de la mélamine (polycondensats), lesdites sections de réaction (R) et de post-réaction et de décapage avec l'ammoniac (PR) sont respectivement reliées, par des conduites (16R, 16P), à la section de récupération de l'ammoniac (AR) d'où elles reçoivent un flux d'ammoniac gazeux, anhydre et surchauffé sous pression (16R) et un flux d'ammoniac gazeux, anhydre et surchauffé sous pression (16P), ladite section de post-réaction (PR) étant reliée par une conduite (4) à la section de trempe-ammonolyse (QAL) dans laquelle elle introduit, par la conduite (4), un flux liquide de mélamine brute appauvrie en CO₂, ladite section de trempe-ammonolyse (QAL) étant reliée, par les conduites (15, 12 et 10), à la section de récupération de l'ammoniac (AR) dont elle reçoit un flux d'ammoniac pur, à la section de traitement de l'eau de traitement (PWT) dont elle reçoit une solution aqueuse d'ammoniac, à une section de séparation (S) dont elle reçoit un flux de liqueur mère, ladite section de trempe-ammonolyse (QAL) étant enfin reliée, par une conduite (5), à une section de purification (P) dans laquelle elle introduit un flux d'ammoniac aqueux comprenant de la mélamine purifiée substantiellement exempte de polycondensats, ladite section de purification (P) étant reliée par une conduite (6) à la section de cristallisation à froid (C) dans laquelle elle introduit une solution de mélamine purifiée, ladite section de cristallisation à froid (C) est reliée par une conduite (7) à une section de séparation (S) dans laquelle elle introduit une suspension de mélamine, ladite section de séparation (S) étant reliée par une conduite (9) à la section de traitement de l'eau de traitement (PWT) dans laquelle elle introduit une première partie de la liqueur mère comprenant de l'H₂O, des traces de mélamine et des OAT à décomposer thermiquement à une température comprise entre 270°C et 300°C, et par une conduite (10) à la section de trempe-ammonolyse (QAL) dans laquelle elle introduit une seconde partie de la liqueur mère comprenant de l'H₂O, des traces de mélamine et des OAT, et, par une conduite (200), à l'extérieur de l'installation où un flux de mélamine pure est envoyé.

11. Installation selon l'une ou plusieurs des revendications 9 et 10, dans laquelle la section de traitement de l'eau de traitement (PWT) est reliée par une conduite (31) à la section de décomposition à basse pression (LPD) dont elle reçoit, par ladite conduite (31), un flux de gaz résiduel comprenant du NH₃, du CO₂ et de l'H₂O, par une conduite (34) à la section de concentration de l'urée (CON) dont elle reçoit un flux aqueux, et par une conduite (300) à l'extérieur de l'installation où un flux d'eau pure est envoyé, ladite section de traitement de l'eau de traitement (PWT) est également reliée, par une conduite (12), à la section de trempe-ammonolyse (QAL) dans laquelle elle introduit un flux liquide comprenant de l'ammoniac et de l'eau, par une conduite (13), à la section de récupération de l'ammoniac (AR) dans laquelle elle introduit un flux liquide comprenant de l'eau, de l'ammoniac et du CO₂ et, par une conduite (14), à la section de condensation de gaz résiduel (OGC) dans laquelle elle introduit un flux gazeux comprenant du NH₃, du CO₂ et de l'H₂O.

12. Installation selon l'une ou plusieurs des revendications 9 à 11, dans laquelle la section de lavage de gaz résiduel (OGQ) est reliée par une conduite (33) à la section de concentration de l'urée (CON) d'où elle reçoit un flux d'urée concentrée et, par une conduite (100), à l'extérieur de l'installation d'où elle reçoit un flux d'urée fondue en provenance des limites de la batterie, ladite section de lavage de gaz résiduel (OGQ) étant ensuite reliée, par une conduite (2), à la section de réaction (R) dans laquelle elle introduit un flux de recyclage de l'urée comprenant de la mélamine retirée du flux de gaz résiduel (23).

13. Installation selon l'une ou plusieurs des revendications 9 à 12, dans laquelle la section de lavage de gaz résiduel (OGQ) est reliée, par une conduite (33), à la section de concentration de l'urée (CON) dont elle reçoit un flux d'urée concentrée, et dans laquelle la section de conversion (OGR) est reliée, par des conduites (101, 102), à l'extérieur de l'installation dont elle reçoit un flux d'ammoniac liquide et un flux de CO₂ gazeux.

14. Installation selon l'une ou plusieurs des revendications 9 à 13, dans laquelle la section de concentration de l'urée (CON) est reliée, par une conduite (110), à l'extérieur de l'installation d'où elle reçoit un flux de solution d'urée provenant des limites de la batterie, et, par une conduite (33), à la section de lavage de gaz résiduel (OGQ) dans laquelle elle introduit un flux d'urée concentrée, ladite section de lavage de gaz résiduel (OGQ) étant ensuite reliée, par une conduite (2), à la section de réaction (R), dans laquelle elle introduit un flux de recyclage de l'urée comprenant la mélamine extraite du flux de gaz résiduel (23).
